# EUROPEAN PATENT APPLICATION

(11) **EP 4 434 995 A1**
(43) Date of publication of application: **25.09.2024**
(21) Application number: 23163747.1
(22) Date of filing: 23.03.2023
(51) Int. Cl.: C07F 15/00, C07F 15/02, C07F 19/00

(54) **CATALYTIC DECARBOXYLATION REACTIONS USING IONIC LIQUIDS**

(71) Applicant: Max-Planck-Gesellschaft zur Förderung der Wissenschaften e.V., 80539 München (DE)
(72) Inventor: Levin Rojas, Natalia, 45470 Mülheim a.d. Ruhr (DE); Goclik, Lisa, 45472 Mülheim a.d. Ruhr (DE); Walschus, Henrik, 47057 Duisburg (DE); Bordet, Alexis, 45479 Mühlheim a.d. Ruhr (DE); Leitner, Walter, 52074 Aachen (DE)
(74) Representative: Hoffmann Eitle

(57) **Abstract**

The present invention is directed towards catalysts comprising ionic liquids, their use in decarboxylation reactions and their use in tandem reduction and decarboxylation reactions. A method employing such catalysts to access phenol and aniline derivatives is also claimed. The catalysts and methods of the present invention improve upon the disadvantages associated with literature decarboxylation reactions.

## Description

### Field of the Invention

The present invention is directed towards a catalyst, its use in decarboxylation reactions and its use in tandem reduction/decarboxylation reactions.

### Background of the Invention

Phenol and its derivatives (e.g., alkylphenols and aminophenols) are important compounds that find widespread application in all areas of the chemical industry. More than 99% of the world's phenol production arises from the cumene process where the petrochemical feedstocks benzene and propene are converted into phenol and acetone. Alkylphenols are typically produced through the direct alkylation of phenol derivates with olefins. A disadvantage of this process is that it suffers from a limited substrate scope. Alternatively, a direct alkylation of phenol through a Friedel-Craft alkylation can be utilised. Disadvantages of this process include harsh conditions and poor chemo- and regioselectivity. Similarly, the selective amination of phenol to produce aminophenols remains a challenge.

Therefore, alternative methods to synthesise phenols are sought-after. For example, the catalytic cleavage of lignin-derived diaryl ethers and lignin model compounds can lead to alkylphenols, but current catalytic systems still suffer from low selectivity, narrow substrate scope, low catalyst stability or poor recyclability.

In addition to phenols and aminophenols, aniline and its alkylated derivatives are widely used in the chemical industry (3.8 million tons in 2008) as a precursor of polyurethanes, rubber additives, dyes and pigments, agricultural chemicals and pharmaceuticals. Aniline, as well as phenol, is commercially produced mostly from the oil-based benzene. The main production route includes nitration of benzene under highly concentrated sulphuric and nitric acid conditions and further catalytic hydrogenation. Derivatization of aniline to alkylanilines is also of commercial relevance and presents different drawbacks. Friedel-Crafts alkylation of aniline under high temperatures and pressures affords ortho alkylanilines and the direct amination of arenes requires multiple reaction steps and harsh reaction conditions. Derivatization of aniline to produce alkylanilines has been the focus of research since long date and continues to be a field of development. Aniline production from anthranilic acid, which can be biosynthesized, is attractive but remains a poorly explored pathway, restricted to strongly acidic conditions.

A possible alternative method of accessing phenols and anilines involves the selective decarboxylation of benzoic acid derivatives. However, aromatic decarboxylation reactions are generally difficult to achieve. This is partly due to unstable intermediates that are formed, resulting in methods requiring harsh, forcing conditions. Conventional methods described in the literature include heating in the presence of a strong acid, heating with a copper catalyst and quinoline, and mercury-mediated decarboxylation reactions (limited by the toxicity of the organomercury(II) compounds). Developments to these procedures will be briefly outlined below.

Decarboxylation of aromatic carboxylic acids is typically performed using molecular transition-metal catalysts based on metals such as Cu, Ag, Au, Pd or Rh in the presence of additives.

J.S. Dickstein, C.A. Mulrooney, E.M. O'Brien, B.J. Morgan and M.C. Kozlowski ("Development of a Catalytic Aromatic Decarboxylation Reaction", Org. Lett., Vol. 9, No. 13, 2007) describe a palladium-catalysed decarboxylation reaction. Drawbacks of these reaction conditions include that they are only reported for ortho-substituted aromatic carboxylic acids, and that as much as 20 mol% of expensive molecular Pd(O₂CCF₃)₂ catalyst are required, which cannot be reused nor recovered through recycling experiments.

L.J. Gooßen, C. Linder, N. Rodriguez, P.P. Lange and Andreas Fromm ("Silver-catalysed protodecarboxylation of carboxylic acids", Chem. Commun., 2009, 7173-7175) describe silver-based catalyst systems to promote the protodecarboxylation of carboxylic acids. Drawbacks include that this method requires the presence of an ortho substituent, high amounts of catalyst (10 mol%), high quantities of additive (15 mol%), results in side-products or impurities from protodemetallation and given the need of molecular catalyst and additives, the reaction is not suited for recyclability of the catalyst.

S. Seo, J.B. Taylor and M.F. Greaney ("Protodecarboxylation of benzoic acids under radical conditions", Chem. Commun., 2012, 48, 8270-8272) describe oxidative radical conditions (using an oxidant and silver) to decarboxylate acids in acetonitrile. Drawbacks include the use of high amounts of catalyst and high amounts of additive, which are both non-recyclable.

In summary, traditional methods of aromatic decarboxylation reactions are coupled with significant drawbacks, including the need for high amounts of additives, narrow range of functional group tolerance, high amounts of catalyst, catalysts are not recyclable, as well as unwanted side-reactions.

Another paper dealing with decarboxylation reactions is ACS Catal. 2021, 11, 14625-14634 (Liu and Lercher et al.; "On the Mechanism of Catalytic Decarboxylation of Carboxylic Acids on Carbon-Supported Palladium Hydride"). This discusses the use of a catalyst pre-treated with hydrogen for the decarboxylation of arylaliphatic acids. Drawbacks include the narrow substrate scope and lack of recyclability of the catalyst.

To avoid the disadvantages associated with past methods, the present inventors have accessed phenols and anilines through the selective decarboxylation of benzoic acid derivatives, including substrates that can be obtained from lignocellulosic biomass (e.g. through oxidative depolymerisation of lignin, biosynthesis, etc.), such as 4-hydroxybenzoic acid, vanillic acid and syringic acid. Furthermore, the present inventors have obtained alkylphenols and aminophenols *via* a tandem combination of selective decarboxylation reactions with reduction reactions (including, e.g., hydrogenation/ hydrodeoxygenation) of various substrates, including but not limited to easily accessible acyl, formyl, and nitro-functionalised hydroxybenzoic acid derivatives.

It is an object of the present invention to access phenol and aniline derivatives and to improve upon the disadvantages associated with conventional methods. In particular, the present invention allows for the production of phenol and aniline derivatives with good yields, good selectivity and good substrate scope, whilst the catalyst according to the present invention is stable and recyclable. Furthermore, only low amounts of catalyst (typically 1-2 mol%) are necessary.

### Summary of the Invention

The above object is solved by a catalyst comprising supported ionic liquids and ionic liquids with a basic group, processes incorporating such a catalyst to access phenol and aniline derivatives and a catalyst for use in decarboxylation and/or tandem decarboxylation/reduction reactions as defined in claims 1, 6, 7, 8, and 11. Preferred embodiments are the subject of the dependent claims.

It is noted that in the context of decarboxylation reactions, to the best of the inventors' knowledge, ionic liquids have only been described on two occasions:
A. Sharma, R. Kumar, N. Sharma, V. Kumar, A.K. Sinha; Adv. Synth. Catal. 2008, 350, 2910-2920: "Unique Versatility of Ionic Liquids as Clean Decarboxylation Catalyst Cum Solvent: A Metal- and Quinoline-Free Paradigm towards Synthesis of Indoles, Styrenes, Stilbenes and Arene Derivatives under Microwave Irradiation in Aqueous Conditions".
D. Liu, J. Sun, B.A. Simmons, S. Singh; ACS Sustainable Chem. Eng. 2018, 6, 7232-7238: "N-Heterocyclic Carbene Promoted Decarboxylation of Lignin-Derived Aromatic Acids".

In both cases only moderate to poor yields were obtained, the reactions were limited to narrow substrate scopes of aromatic carboxylic acids and the decarboxylation reactions are metal-free and hydrogen-free.

By employing a catalyst according to the present invention, the inventors have improved upon the drawbacks associated with traditional methods of aromatic decarboxylation reactions.

### Brief Description of the Drawings

Figure 1: Overview of methods for covalent attachment of a linker group to a support. Silanization (Fig. 1A), amidation (Fig. 1B) and C-C coupling (Fig. 1B and Fig. 1C).
Figure 2: Overview of catalysts according to the present invention.
Figure 3: Overview of method of decarboxylation reaction according to the invention
Figure 4: Overview of method of tandem reduction and decarboxylation reaction according to the invention.
Figure 5: Measurements taken for the Fe₂₅Ru₇₅-SILP^{A} + IL-NEt₂ catalyst. Figure 5a is a representation/illustration of the bimetallic catalyst. Figure 5b is a STEM-HAADF image. Figure 5c is a STEM-HAADF-EDS elemental mapping.
Figure 6: ¹H NMR (400 MHz, acetone-d4) spectra of a) phenol, b) product, isolated from applying phenol to decarboxylation conditions under D₂, and c) product, isolated from decarboxylation of 4-hydroxybenzoic acid under D₂.
Figure 7: NMR (400 MHz, acetone-d6) spectra of a 1 h reaction of 4-hydroxybenzoic acid under 20 bar D₂ at 200 °C. a) ¹H NMR spectrum, b) zoom to the aromatic area of a), c) ²H NMR spectrum, d) ¹³C NMR spectrum.
Figure 8: Time profile of reduction and decarboxylation of 3-nitro-4-hydroxybenzoic acid (**12**) using Fe₂₅Ru₇₅-SILP^{A}+IL-NEt₂. Conditions: Catalyst (10 mg), substrate: 24.2 mg (30 eq. related to amine, 65 eq. related to total metal loading), 0.5 mL DMF, 50 bar H₂, 150 °C, 500 rpm. Y = Yield, determined by GD-FID using tetradecane as internal standard. Error bars represent the standard deviation for two experiments.
Figure 9: Catalyst recycling, decarboxylation of 4-hydroxybenzoic acid (**1**). Conditions: Fe₂₅Ru₇₅-SILP^{A}+IL-NEt₂ (20 mg), substrate: 30.6 mg (15 eq. related to amine), 1 mL mesitylene, 50 bar H₂, 200 °C, 6 h, 500 rpm. Product yields determined by GD-FID using tetradecane as internal standard. Selectivity toward **1a** >99%.
Figures 10-17: NMR characterisation associated to isolated products.

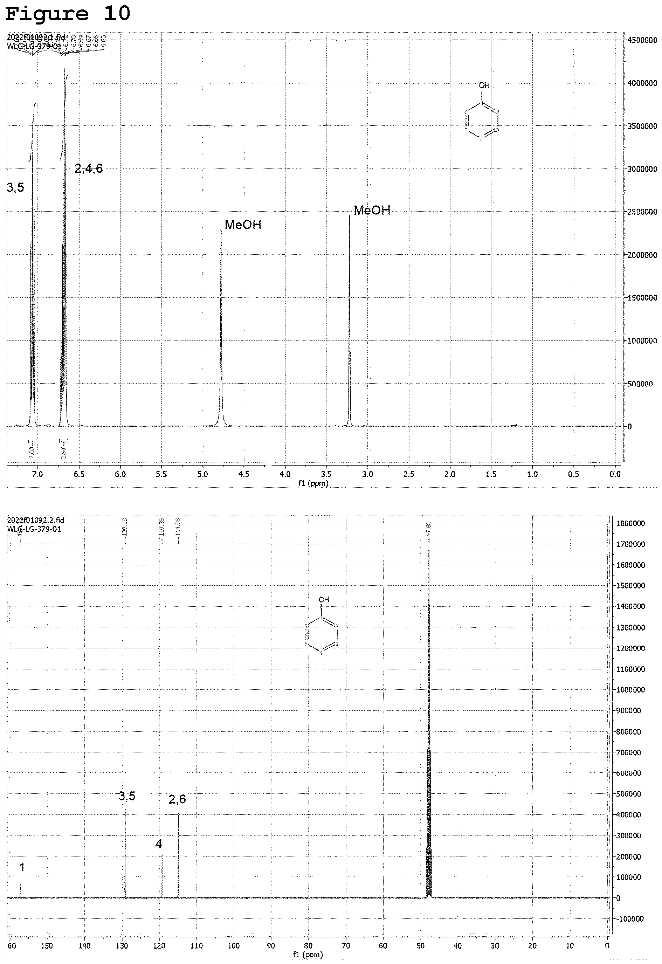

   Figure 10: Phenol (1a)
      ¹H NMR (400 MHz, Methanol-*d*₄) *δ* (ppm) = 7.11 - 7.02 (m, 2H), 6.74 - 6.64 (m, 3H).
      ¹³C{¹H} NMR (100 MHz, Methanol-*d*₄) *δ* (ppm) = 157.23 (1C), 129.19 (2C), 119.26 (1C), 114.98 (2C).

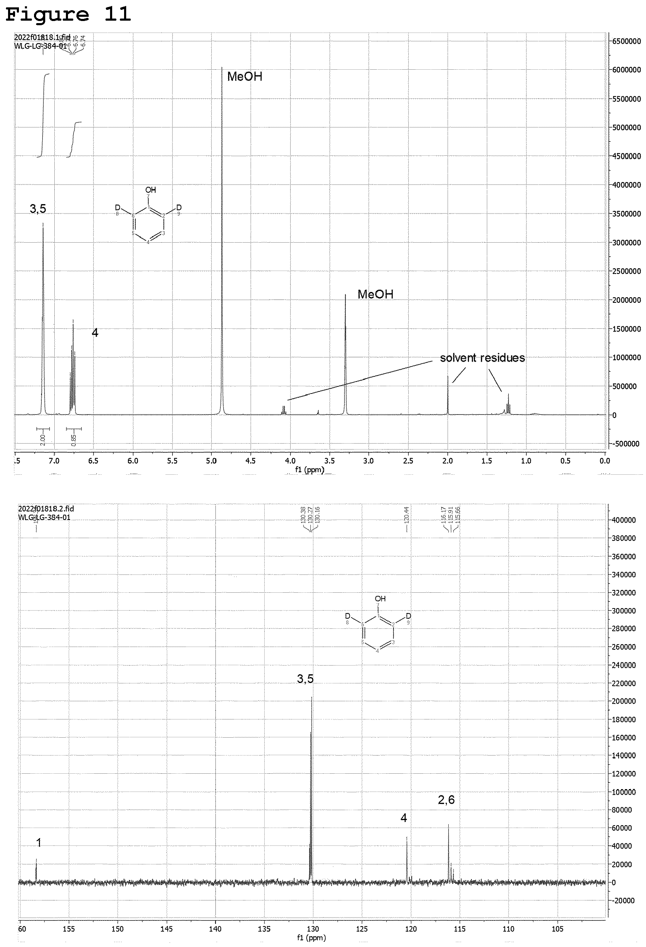

   Figure 11: Product, isolated from application of phenol to decarboxylation conditions under D₂
      ¹H NMR (400 MHz, Methanol-*d*₄) *δ* (ppm) = 7.14 (s, 2H), 6.85 - 6.65 (m, 1H).
      ¹³C{¹H} NMR (100 MHz, Methanol-*d*₄) *δ* (ppm) = 158.34 (1C), 130.27 (2C), 120.44 (1C), 115.91 (2C).

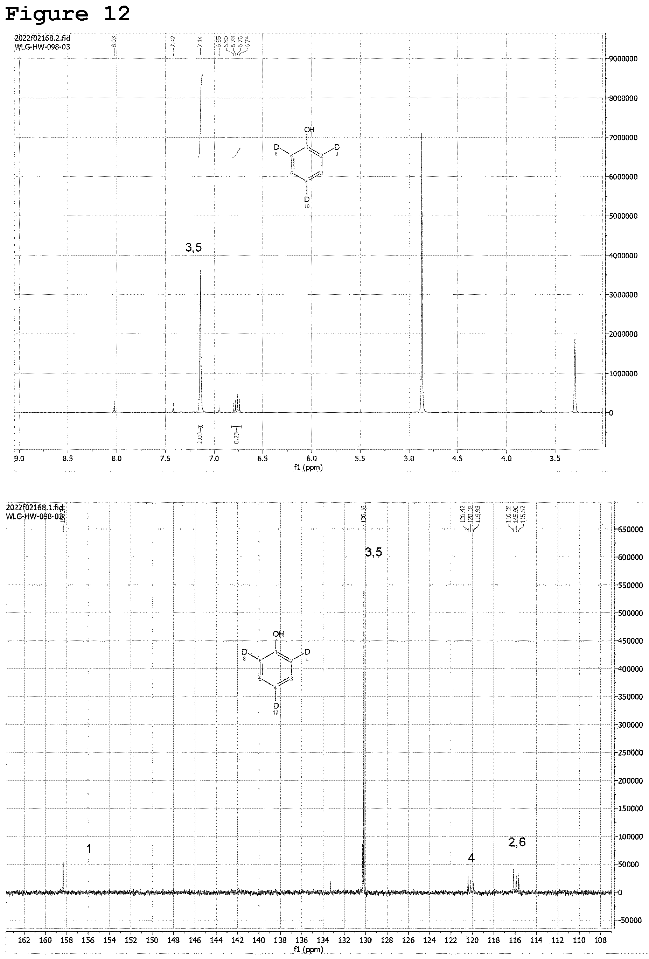

   Figure 12: Product, isolated from decarboxylation of 4-hydroxybenzoic acid under D₂
      ¹H NMR (400 MHz, Methanol-*d*₄) *δ* (ppm) = 7.14 (s, 2H) .
      ¹³C NMR (100 MHz, Methanol-*d*₄) *δ* (ppm) = 158.34 (1C), 130.16 (2C), 120.18 (1C), 115.90 (2C).

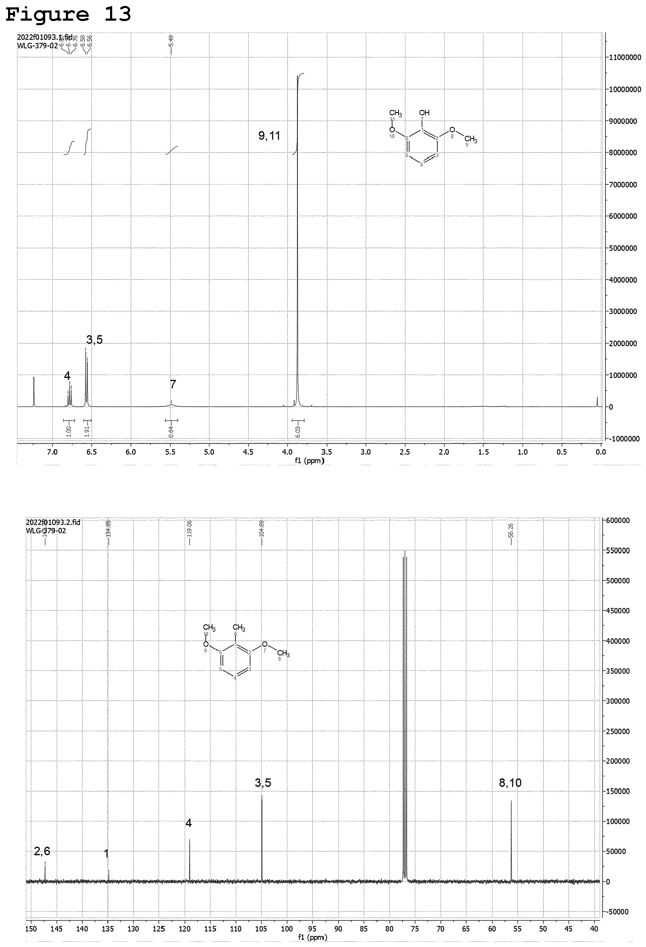

   Figure 13: 2,6-Dimethoxyphenol (3a)
      ¹H NMR (400 MHz, Chloroform-d) *δ* (ppm) = 6.86 - 6.72 (m, 1H), 6.57 (d, *J* = 8.3 Hz, 2H), 5.49 (s, 1H), 3.87 (s, 6H).
      ¹³C{¹H} NMR (100 MHz, Chloroform-*d*) *δ* (ppm) = 147.25 (2C), 134.85 (1C), 119.06 (1C), 104.89 (2C), 56.26 (2C).

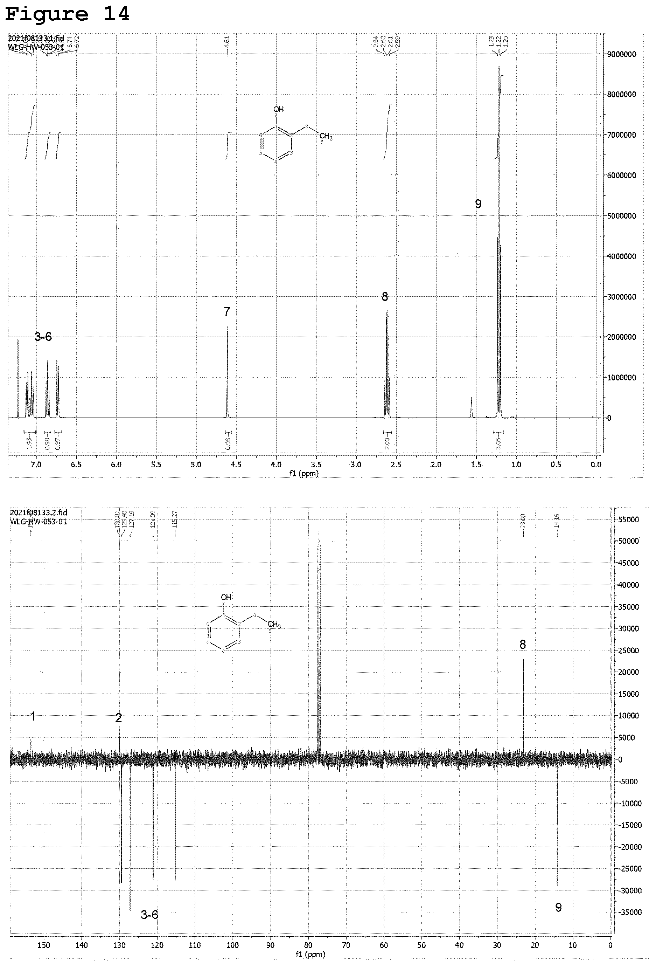

   Figure 14: 2-Ethylphenol (10a)
      ¹H NMR (400 MHz, Chloroform-*d*) *δ* (ppm) = 7.15 - 7.01 (m, 2H), 6.89 - 6.82 (m, 1H), 6.73 (d, *J* = 8.0 Hz, 1H), 4.61 (s, 1H), 2.62 (q, *J* = 7.6 Hz, 2H), 1.22 (t, *J* = 7.6 Hz, 3H).
      ¹³C (APT) NMR (100 MHz, Chloroform-*d*) *δ* (ppm) = 153.46 (1C), 130.01 (1C), 129.48 (1C), 127.19 (1C), 21.09 (1C), 115.27 (1C), 23.09 (1C), 14.16 (1C).

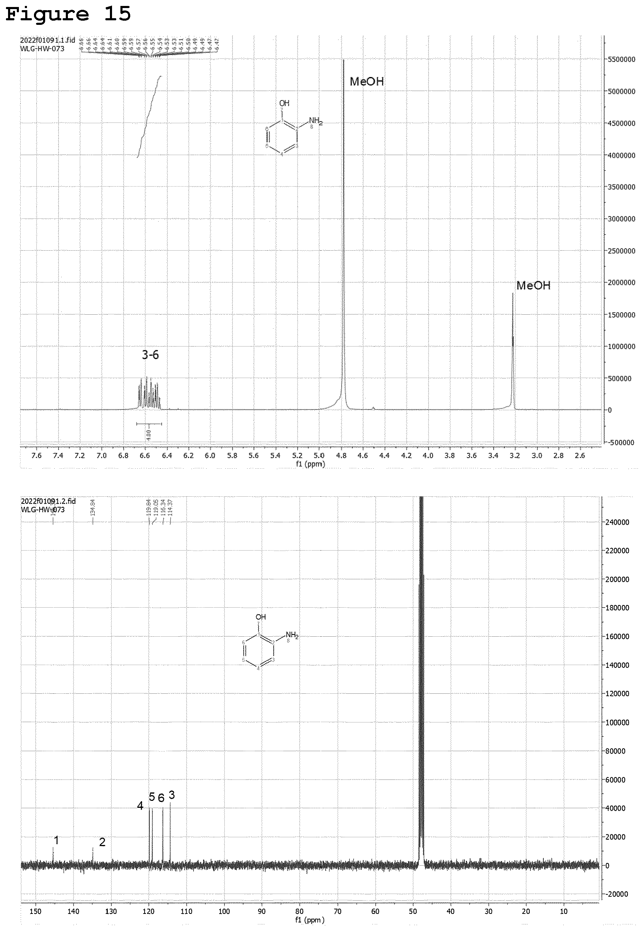

   Figure 15: 2-Aminophenol (6a)
      ¹H NMR (400 MHz, Methanol*-*d₄) *δ* (ppm) = 6.66 - 6.47 (m, 4H).
      ¹³C{¹H} NMR (100 MHz, Methanol-d₄) *δ* (ppm) = 145.38 (1C), 134.84 (1C), 119.84 (1C), 119.05 (1C), 116.34 (1C), 114.37 (1C).

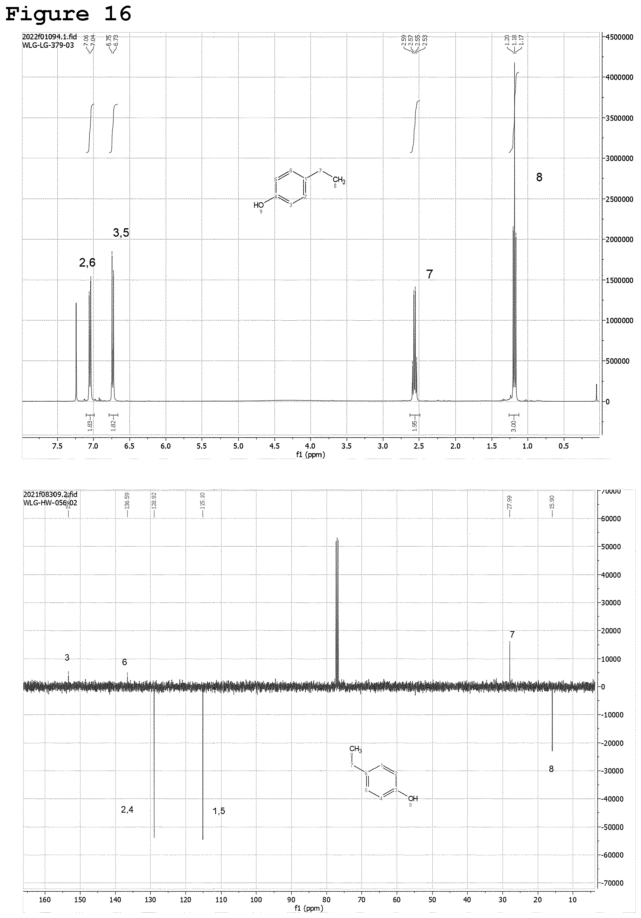

   Figure 16: 4-Ethylphenol (13a)
      ¹H NMR (400 MHz, Chloroform-*d*) *δ* (ppm) = 7.05 (d, *J* = 8.5 Hz, 2H), 6.74 (d, *J* = 8.5 Hz, 2H), 2.56 (q, J = 7.6 Hz, 2H), 1.18 (t, *J* = 7.6 Hz, 3H).
      ¹³C (APT) NMR (100 MHz, Chloroform-*d*) *δ* (ppm) = 153.38 (1C), 136.59 (1C), 128.92 (2C), 115.10 (2C), 27.99 (1C), 15.90 (1C).

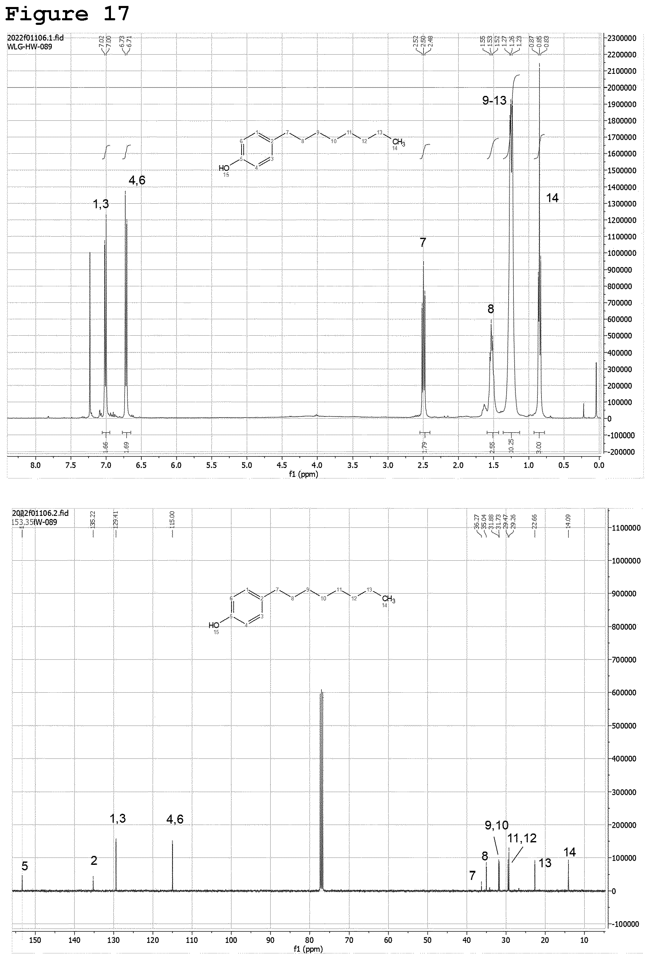

   Figure 17: 4-Octylphenol (14a)
      ¹H NMR (400 MHz, Chloroform-*d*) *δ* (ppm) = 7.01 (d, *J* = 8.3 Hz, 2H), 6.72 (d, *J* = 8.4 Hz, 2H), 2.55 - 2.40 (m, 2H), 1.59 - 1.43 (m, 2H), 1.37 - 1.13 (m, 10H), 0.85 (t, *J* = 6.8 Hz, 3H).
      ¹³C{¹H} NMR (100 MHz, Chloroform-*d*) *δ* (ppm) = 153.35 (1C), 135.22 (1C), 129.41 (2C), 115.00 (2C), 26.27 (1C), 35.04 (1C), 31.88 (1C), 31.73 (1C), 29.47 (1C), 29.36 (1C), 22.66 (1C), 14.09 (1C).

### Detailed Description

Embodiments according to the present invention will now be described in more detail.

As used herein, an "ionic liquid" (IL) is a salt in the liquid state at or below 100 °C at atmospheric pressure, i.e., the pure IL is a liquid and contains a mixture of cations and anions in these conditions. The ionic liquids according to the present invention are defined by reference to the structures below. Preferably, the ionic liquids according to the invention are as described in claim 1.

As used herein, the term "support" (Su) refers to a solid material on which the components of the catalyst are immobilised.

As used herein, the term "linker group" (L) refers to a molecular functionality that is responsible for the attachment of the molecular modifier to the support.

As used herein, the term "spacer group" (Sp) refers to a molecular group that provides separation and connection between the linker and the cation.

As used herein, the term "cation" (C) refers to a positively charged molecular group that is part of the IL. That is, (C) (and likewise C¹ and C²) as used in the present invention refers to a cationic group covalently bonded within the IL.

As used herein, the term "anion" (A) refers to a negatively charged molecular group that is part of the IL. The anion (A) (and likewise A¹ and A²), which may be one or more anions, is the counterion of the cation (C). That is, the one or more anions can compensate for the positive charge(s) of cation (C), i.e., the cationic group within the structure of the IL.

As used herein, the term "bimetallic nanoparticles" refers to nanoparticles comprised of two different metals.

As used herein, the term "3d transition metal" refers to metals from the first transition series or 3d series, including Sc, Ti, V, Cr, Mn, Fe, Co, Ni, Cu and Zn.

As used herein, the term "noble metal" refers to metals that have outstanding resistance to oxidation, including Rh, Ru, Pd, Pt, Ag, Re, Os, Ir and Au.

As used herein, the term "tandem" refers to both a decarboxylation and a reduction reaction taking place on the same substrate.

As used herein, the term "supported ionic liquid phase" (SILP) refers to an ionic liquid structure attached to a support.

In the SILP, a molecular modifier (ionic liquid-like structure) is chemisorbed on a support. In other words, the molecular modifier is covalently attached to the support.

The supported ionic liquid phase for use in the present invention can have the general structure:

Su - L - Sp - C¹ - alkylene group - CH₃ or D

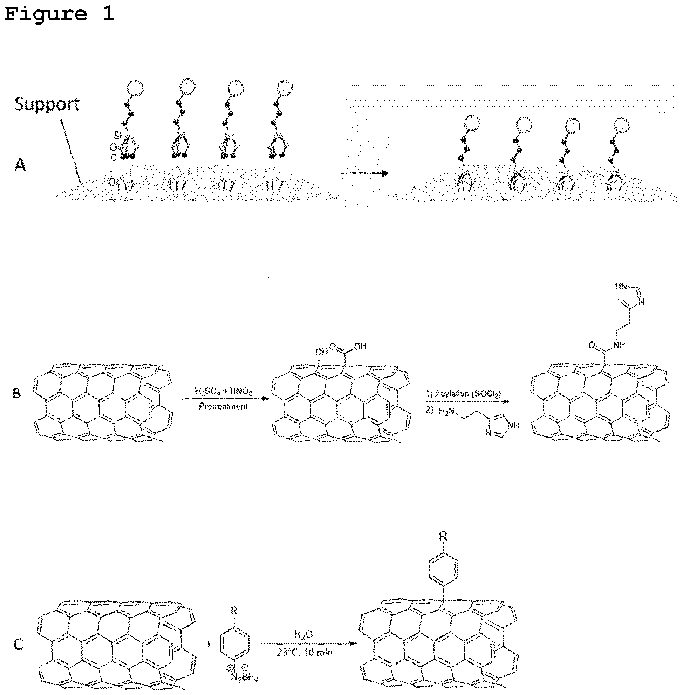

wherein Su is a support, L is a linker group, Sp is a spacer group, C¹ is a cation, D is a basic group, and further comprising a counterion A¹ which is an anion. The catalyst can optionally contain bimetallic nanoparticles that are immobilised on the support. The covalent attachment of the linker group to the support can proceed through different methods. Examples of such methods include silanization (Fig. 1A), amidation (Fig. 1B) and C-C coupling (Fig. 1B and Fig. 1C) .

Silanization is the preferred method for metal oxide supports. For chemisorption of molecular modifiers on carbon-based support materials, amidation or C-C coupling are preferred methods. The carbon-based support can be activated by generation of -COOH functionalities on the surface and then reacted with amines to form amide bonds. Alternatively, direct C-C coupling can be achieved using diazonium chemistry. In this latter case, the linker can be an arene group.

When the molecular modifier is attached on the support via silanization, the linker group is an -Si-O- group. When the molecular modifier is attached on the support via C-C coupling, the linker group is a C-C linkage, wherein the C-C linkage is preferably an alkylene or arylene group. When the molecular modifier is attached on the support via amidation, the linker group is an amide group.

The catalyst for use in the present invention can involve i) a supported ionic liquid phase and ii) a further ionic liquid with basic groups. The catalyst can optionally contain bimetallic nanoparticles that are immobilised on the support.

The further ionic liquid with basic groups is physisorbed to the support. That is, the further ionic liquid with basic groups is not covalently attached to the support. Examples of how ionic liquids can be physisorbed to a support include the stabilisation of an ionic liquid through the formation of non-covalent bonds such as dispersion, electrostatic, or van der Waals interactions. An example in the literature for the physisorption of ionic liquids to the support can be found in Offner-Marko, L.; Bordet, A.; Moos, G.; Tricard, S.; Rengshausen, S.; Chaudret, B.; Luska, K. L.; Leitner, W. "Bimetallic Nanoparticles in Supported Ionic Liquid Phases as Multifunctional Catalysts for the Selective Hydrodeoxygenation of Aromatic Substrates" Angew. Chemie - Int. Ed. 2018, 57 (39), 12721-12726.

An ionic liquid which is physisorbed to the support for use in the present invention can have the general structure:

CH₃ - alkylene group - C² - alkylene group - D

wherein C² is a cation, D is a basic group; and further comprising a counterion A² which is an anion.

Catalysts including ionic liquids have recently attracted attention in the use of hydrogenation reactions and are generally described in the following papers:
Luska, K. L.; Julis, J.; Stavitski, E.; Zakharov, D. N.; Adams, A.; Leitner, W. "Bifunctional Nanoparticle-SILP Catalysts (NPs@SILP) for the Selective Deoxygenation of Biomass Substrates" Chem. Sci. 2014, 5, 4895-4905. This paper describes the synthesis of a catalyst comprised of Ru nanoparticles (NPs) immobilized on an acid-functionalized SILP and its use for deoxygenation reactions.
Luska, K. L.; Bordet, A.; Tricard, S.; Sinev, I.; Grünert, W.; Chaudret, B.; Leitner, W. "Enhancing the Catalytic Properties of Ruthenium Nanoparticle-SILP Catalysts by Dilution with Iron" ACS Catal. 2016, 6, 3719-3726. This article describes the preparation of bimetallic FeRu NPs supported on a SILP without acid or basic function and its use for hydrogenation reactions.
Offner-Marko, L.; Bordet, A.; Moos, G.; Tricard, S.; Rengshausen, S.; Chaudret, B.; Luska, K. L.; Leitner, W. "Bimetallic Nanoparticles in Supported Ionic Liquid Phases as Multifunctional Catalysts for the Selective Hydrodeoxygenation of Aromatic Substrates" Angew. Chemie - Int. Ed. 2018, 57, 12721-12726. This paper describes the synthesis of FeRu NPs supported on an acid-functionalized SILP and its use for hydrodeoxygenation reactions.
Rengshausen, S.; Etscheidt, F.; Großkurth, J.; Luska, K.; Bordet, A.; Leitner, W. "Catalytic Hydrogenolysis of Substituted Diaryl Ethers by Using Ruthenium Nanoparticles on an Acidic Supported Ionic Liquid Phase (Ru@SILP-SO3H)" Synlett 2019, 30, 405-412. This article describes the synthesis of Ru NPs immobilized on an acid-functionalized SILP and its use for hydrogenolysis reactions.
Bordet, A.; Moos, G.; Welsh, C.; Licence, P.; Luska, K. L.; Leitner, W. "Molecular Control of the Catalytic Properties of Rhodium Nanoparticles in Supported Ionic Liquid Phase (SILP) Systems" ACS Catal. 2020, 10, 13904-13912. Moos, G.; Emondts, M.; Bordet, A.; Leitner, W. "Selective Hydrogenation and Hydrodeoxygenation of Aromatic Ketones to Cyclohexane Derivatives Using a Rh@SILP Catalyst" Angew. Chemie Int. Ed. 2020, 59, 11977-11983. These papers report the preparation of Rh NPs immobilized on different SILPs and its use for hydrogenation and hydrodeoxygenation reactions.
Goclik, L.; Offner-Marko, L.; Bordet, A.; Leitner, W. "Selective Hydrodeoxygenation of Hydroxyacetophenones to Ethyl-Substituted Phenol Derivatives Using a FeRu@SILP Catalyst" Chem. Commun. 2020, 56, 9509-9512. Goclik, L.; Walschus, H.; Bordet, A.; Leitner, W. "Selective Hydrodeoxygenation of Acetophenone Derivatives Using a Fe25Ru75@SILP Catalyst: A Practical Approach to the Synthesis of Alkyl Phenols and Anilines" Green Chem. 2022, 24, 2937-2945. These papers report the use of FeRu NPs on a SILP without basic or acid functions for hydrodeoxygenation reactions.
Kacem, S.; Qiao, Y.; Wirtz, C.; Theyssen, N.; Bordet, A.; Leitner, W. "Supercritical Carbon Dioxide as Reaction Medium for Selective Hydrogenation of Fluorinated Arenes" Green Chem. 2022, 24, 8671-8676. This paper describes the preparation of Rh NPs immobilized on silica modified with decyl chains and its use for hydrogenation reactions.
Rengshausen, S.; Van Stappen, C.; Levin, N.; Tricard, S.; Luska, K. L.; DeBeer, S.; Chaudret, B.; Bordet, A.; Leitner, W. "Organometallic Synthesis of Bimetallic Cobalt-Rhodium Nanoparticles in Supported Ionic Liquid Phases (Co x Rh 100-x @SILP) as Catalysts for the Selective Hydrogenation of Multifunctional Aromatic Substrates" Small 2021, 17, 2006683. This paper reports the use of CoRh NPs on a SILP without basic or acid functions and its use for hydrogenation reactions.
Sisodiya-Amrute, S.; Van Stappen, C.; Rengshausen, S.; Han, C.; Sodreau, A.; Weidenthaler, C.; Tricard, S.; DeBeer, S.; Chaudret, B.; Bordet, A.; Leitner, W. "Bimetallic MxRu100-x Nanoparticles (M = Fe, Co) on Supported Ionic Liquid Phases (MxRu100-x@SILP) as Hydrogenation Catalysts: Influence of M and M:Ru Ratio on Activity and Selectivity" J. Catal. 2022, 407, 141-148. This paper reports the use of FeRu and CoRu NPs on a SILP without basic or acid functions for hydrogenation reactions.
Louis Anandaraj, S. J.; Kang, L.; DeBeer, S.; Bordet, A.; Leitner, W. "Catalytic Hydrogenation of CO 2 to Formate Using Ruthenium Nanoparticles Immobilized on Supported Ionic Liquid Phases" Small 2023, 2206806. This paper reports the use of Ru NPs on a SILP without basic or acid functions for hydrogenation reactions.

However, to the best of the inventors' knowledge, such catalysts have not been employed in decarboxylation reactions.

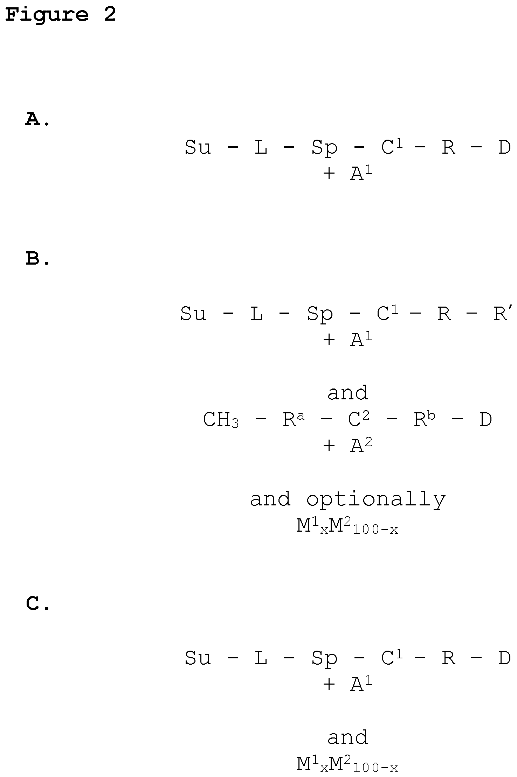

According to an embodiment of the present invention (Figure 2A), a catalyst comprises the following structure:

Su - L - Sp - C¹ - R - D

wherein:
Su is a support;
L is a linker group;
Sp is a spacer group;
C¹ is a cation;
R is an alkylene group with 1-20 carbon atoms, optionally containing one or more groups selected from ether, ester, amido or arylene groups, and/or wherein the alkylene group is optionally substituted with an aryl group; and D is a basic group;
wherein the structure further comprises a counterion A¹, which is an anion.
The definitions of the following embodiment equally apply to the above.

According to a further embodiment of the present invention (Figure 2B), a catalyst comprises:
(i) the following structure **I**:

   Su - L - Sp - C¹ - R - R'

   wherein:
   Su is a support;
   L is a linker group;
   Sp is a spacer group;
   C¹ is a cation;
   R is an alkylene group with 1-20 carbon atoms, optionally containing one or more groups selected from ether, ester, amido or arylene groups; and R' is -CH₃ or D, wherein D is a basic group;
   wherein structure I further comprises a counterion A¹ which is an anion;
      and
(ii) the following structure **II**:

   CH₃ - R^{a} - C² - R^{b} - D

   wherein R^{a} and R^{b} are each independently an alkylene group with 1-20 carbon atoms, optionally containing one or more groups selected from ether, ester, amido or arylene groups; C² is a cation; and D is a basic group;
   wherein structure **II** further comprises a counterion A² which is an anion.

The basic groups in structure **I** and in structure **II** can be the same, or they can be different.

The alkylene group in R can be further substituted with an aryl group.

In a preferred embodiment, R, R^{a} and R^{b} are each independently an alkylene group with 1-10 carbon atoms, more preferably 1-6 carbon atoms, most preferably 2-4 carbon atoms.

In a preferred embodiment, the catalyst further comprises bimetallic nanoparticles that are immobilised on the support Su, wherein the bimetallic nanoparticles are represented by:

M¹ₓM²₁₀₀₋ₓ

wherein:
M¹ is a 3d transition metal;
M² is a noble metal; and
x is a number between 1 and 99, preferably between 10 and 80.

As will be appreciated, the subscript x as used herein in the definition of the bimetallic nanoparticles denotes the relative, preferably average, atomic proportions of M¹ and M² in the bimetallic nanoparticles.

The spacer (Sp) can be an alkylene group with 1-30 carbon atoms, optionally containing one or more groups selected from ether, ester, amido or arylene groups. Preferably, the spacer Sp is an alkylene group with 2-10 carbon atoms, most preferably with 2-4 carbon atoms.

The support (Su) can be selected from SiO₂, Al₂O₃, TiO₂, ZrO₂, CaO₂, ZnO₂, MgO₂, CeO₂, graphene, graphitic material, activated charcoal or carbon nanotubes. Preferably, the support is SiO₂.

The linker (L) can be an -Si-O- group, a C-C linkage or an amido group connected to the support. Preferably, the linker is a -Si-O- group connected to the support. When the linker is an -Si-O- group, the spacer is preferably an alkylene group with 1-30 carbon atoms. A C-C linkage can be an alkylene or an arylene group.

C¹ and C² can each independently be imidazolium, phosphonium, pyridinium, ammonium, or triazolium. Preferably C¹ and C² are the same, more preferably C¹ and C² are imidazolium. C¹ and C² being the same has the advantage that leaching of the ionic liquid is reduced.

A¹ and A² can each independently be NTf₂⁻, OTf⁻, CTf₃⁻, halides, OAc⁻, HCO₂⁻, HCO₃⁻, CO₃²⁻, NO₃⁻, BF₄⁻, PF₆⁻, CH₃SO₃⁻, CF₃SO⁻, perfluorobutanesulfonate, perfluorooctanoate, bis[(trifluoromethyl)sulfonyl]imide, bis[(pentafluoroethyl)sulfonyl]imide, tris(pentafluoroethyl)trifluorophosphate, imidazolate, dimethylphosphate, or diethylphosphate. Preferably A¹ and A² are the same, more preferably A¹ and A² are NTf₂⁻.

The basic group (D) can be a basic group containing one or more nitrogen atoms. Examples of such basic groups include an amine, an imine and an amidine. Preferably, the basic group is an amine. More preferably, the amine is -NR¹⁴R¹⁵, wherein R¹⁴ and R¹⁵ are each independently H, methyl, ethyl, propyl, or butyl. Even more preferably R¹⁴ and R¹⁵ are ethyl.

M¹ can be Mn, Fe, Co, Ni or Cu. M² can be Rh, Ru, Pd or Pt. Preferably, M¹ is Fe and M² is Ru.

In a preferred embodiment, x is a number between 20 and 60. More preferably, x is a number between 25 and 50.

In a preferred embodiment according to the present invention:
when M¹ is Fe and M² is Ru, x is a number between 1 and 40, preferably a number between 10 and 25, most preferably wherein x is 25;
when M¹ is Co and M² is Ru, x is a number between 50 and 70;
when M¹ is Co and M² is Rh, x is a number between 30 and 80.

The above definitions and preferred embodiments, equally apply to a catalyst according to another embodiment (Figure 2C), wherein the catalyst comprises:
(i) the following structure:

   Su - L - Sp - C¹ - R - D

   wherein:
   Su is a support;
   L is a linker group;
   Sp is a spacer group;
   C¹ is a cation; and
   R is an alkylene group with 1-20 carbon atoms,
   optionally containing one or more groups selected from ether, ester, amido or arylene groups; and
   D is a basic group;
   wherein the structure further comprises a counterion A¹ which is an anion;
      and
(ii) bimetallic nanoparticles immobilised on the support.

The catalysts according to the present invention are illustrated in Figures 2A-2C, wherein Su, L, Sp, C¹, C², A¹, A², R, D, R', R^{a}, R^{b}, M¹, M², and x have the same meanings as described above.

Advantages of the catalysts described include their recyclability and re-usability.

In another embodiment according to the present invention, the catalysts described above can be used in a decarboxylation reaction.

In yet another embodiment according to the present invention, the catalysts described above can be used in a tandem reduction and decarboxylation reaction. Preferably, the reduction is a hydrodeoxygenation.

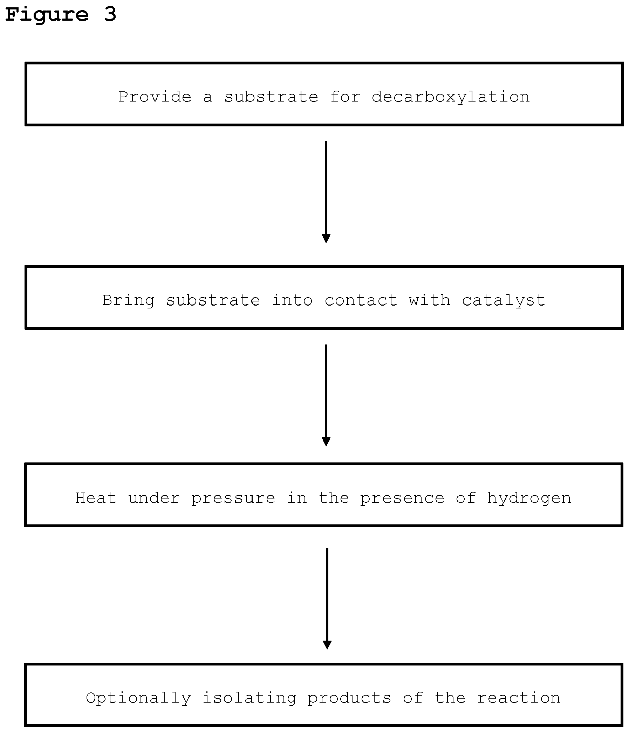

In the following embodiments, methods of decarboxylating substrates are described (Figure 3). These methods result in the production of phenols and/or anilines.

According to an embodiment, a method of decarboxylating a substrate according to formula **IIIa, IIIb, IIIc, IIId,** or **IIIe,** comprises bringing the substrate into contact with a catalyst comprising an ionic liquid with a basic group, and heating under pressure in the presence of hydrogen 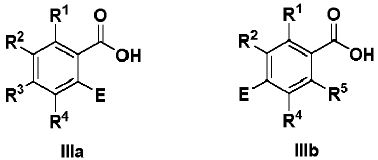 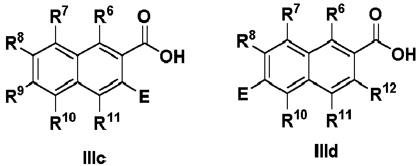 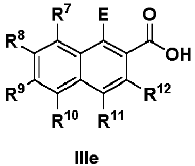 wherein:
E is -OH, -NH₂ or -NR¹³₂, wherein R¹³ is an alkyl group with 1-6 carbon atoms, preferably R¹³ is 1;
   preferably wherein E is -OH, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
   R¹⁶ is an alkyl group with 1-30 carbon atoms, preferably 1-10 carbon atoms;
   the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
   the linear or branched alkyl group contains at least one group selected from C-C double bond, C-C triple bond, keto group, ester group, ether group or amido group;
   preferably R¹ to R¹² are each independently -H, -OMe, -Me, -F, -NH₂, -OH, -NO₂, -COCH₃, -CHO, or - CO-(CH₂)₆-CH₃.

The basic group in the catalyst for use in the above decarboxylation may be a group D as defined for the catalyst according to the present invention, e.g., in claim 4. In a preferred embodiment, the catalyst in the above method comprises bimetallic nanoparticles.

In a preferred embodiment, the substrate is a substrate according to formula **IIIa** or **IIIb**, wherein:
E is -OH or -NH₂; and
R¹ to R⁴ are each independently -H, -OMe, -Me, -F, -NH₂, -OH, -NO₂, -COCH₃, -CHO, or -CO-(CH₂)₆-CH₃.

The inventors unexpectedly found that the presence of molecular hydrogen benefited the decarboxylation reaction using a catalyst comprising an ionic liquid. In the absence of metallic nanoparticles, the presence of hydrogen is beneficial to achieve good mass balances. In the presence of metallic nanoparticles, the decarboxylation mechanism is unusual, since H₂ from the gas phase is activated by the NPs and involved in the protodecarboxylation process. This results in a more effective decarboxylation reaction.

In another embodiment according to the invention, a method of decarboxylation of a substrate according to formula **IIIa, IIIb, IIIc, IIId,** or **IIIe** is described, wherein the method comprises bringing the substrate into contact with a catalyst according to any of the previous embodiments, and heating under pressure in the presence of hydrogen 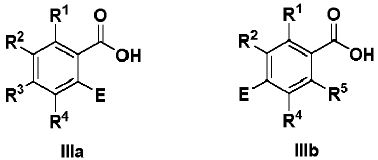 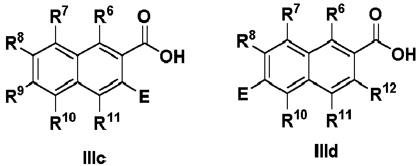 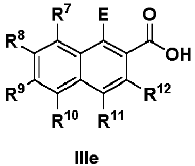 wherein:
E is -OH, -NH₂ or -NR¹³₂, wherein R¹³ is an alkyl group with 1-6 carbon atoms, preferably R¹³ is 1;
   preferably wherein E is -OH, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
   R¹⁶ is an alkyl group with 1-30 carbon atoms, preferably 1-10 carbon atoms;
   the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
   the linear or branched alkyl group contains at least one group selected from C-C double bond, C-C triple bond, keto group, ester group, ether group or amido group;
   preferably R¹ to R¹² are each independently -H, -OMe, -Me, -F, -NH₂, -OH, -NO₂, -COCH₃, -CHO, or - CO- (CH₂)₆-CH₃.

In a preferred embodiment, the substrate is a substrate according to formula **IIIa** or **IIIb,** wherein:
E is -OH or -NH₂; and
R¹ to R⁴ are each independently -H, -OMe, -Me, -F, -NH₂, -OH, -NO₂, -COCH₃, -CHO, or -CO-(CH₂)₆-CH₃.

According to another embodiment of the invention, a method comprising decarboxylation of a substrate according to formula **IIIa, IIIb, IIIc, IIId,** or **IIIe** is described, wherein the method comprises bringing the substrate into contact with a catalyst comprising bimetallic nanoparticles and the following structure, and heating under pressure in the presence of hydrogen and an additive, wherein the additive is a base:

Su - L - Sp - C¹ - R - CH₃

wherein Su is a support; L is a linker group; Sp is a spacer group; C¹ is a cation; and R is an alkylene group with 1-20 carbon atoms, optionally containing one or more groups selected from ether, ester, amido or arylene groups;
wherein the structure further comprises a counterion A¹ which is an anion.

Preferably, the additive is triethylamine or dimethylaniline.

Preferably, the bimetallic nanoparticles that are immobilised on the support are represented by:

M¹ₓM²₁₀₀₋ₓ

wherein: M¹ is a 3d transition metal; M² is a noble metal; and x is a number between 1 and 99, preferably between 10 and 80.

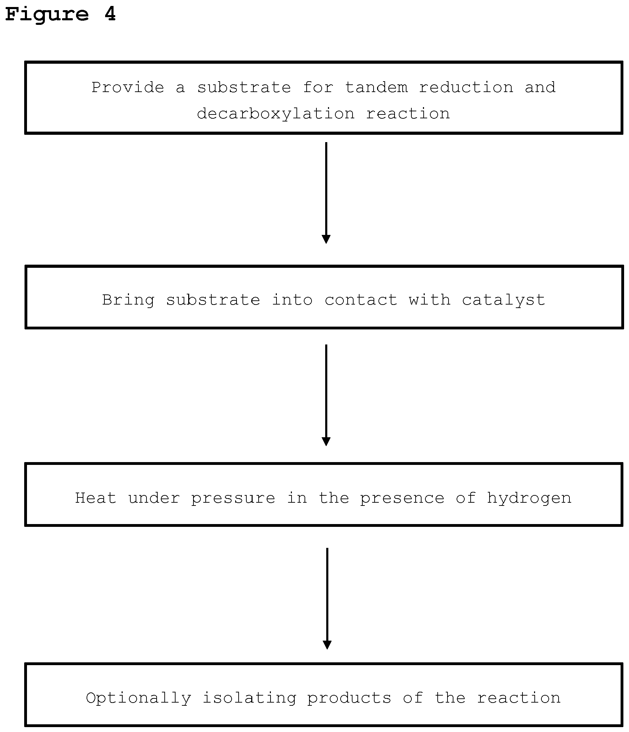

In the following embodiments, methods of tandem decarboxylation and reduction of substrates are described (Figure 4). These methods result in the production of phenols and/or anilines.

According to an embodiment of the invention, a method comprising a tandem decarboxylation and reduction of a substrate according to formula **IIIa, IIIb, IIIc, IIId,** or **IIIe** is described, wherein the method comprises bringing the substrate into contact with a catalyst comprising metal nanoparticles and an ionic liquid with a basic group, and is heated under pressure in the presence of hydrogen 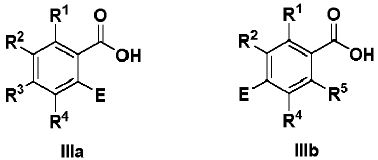 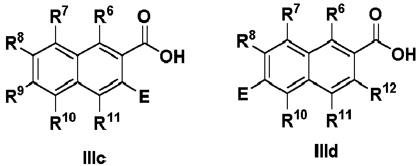 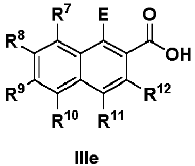 wherein:
E is -OH, -NO₂, -NH₂ or -NR¹³₂, wherein R¹³ is an alkyl group with 1-4 carbon atoms, preferably R¹³ is 1;
   preferably wherein E is -OH, -NO₂, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
   R¹⁶ is an alkyl group with 1-30 carbon atoms, preferably 1-10 carbon atoms;
   the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
   the linear or branched alkyl group contains at least one group selected from C-C double bond, C-C triple bond, keto group, ester group, ether group or amido group;
   preferably R¹ to R¹² are each independently -H, -OMe, -Me, -F, -NH₂, -OH, -NO₂, -COCH₃, -CHO, or - CO- (CH₂)₆-CH₃.

The basic group in the catalyst for use in the above tandem decarboxylation and reduction may be a group D as defined for the catalyst according to the present invention, e.g., in claim 4. In a preferred embodiment, the metal nanoparticles are bimetallic nanoparticles.

In a preferred embodiment, the substrate is a substrate according to formula **IIIa** or **IIIb,** and wherein:
E is -OH, -NO₂ or -NH₂; and
R¹ and R⁴ are each independently -H, -OMe, -Me, -F, -NH₂, -OH, -NO₂, -COCH₃, -CHO, or -CO-(CH₂)₆-CH₃.

The inventors unexpectedly found that the presence of hydrogen (H₂) benefited the tandem reduction and decarboxylation reaction using a catalyst with an ionic liquid. In this case, the effect of the presence of hydrogen is double. On one hand, gaseous H₂ is activated by the metal NPs and involved in the protodecarboxylation process, accelerating the decarboxylation reaction. On the other hand, the activated hydrogen can be used in the reduction of the reducible moieties present in the substrate, leading to the tandem reduction/decarboxylation reactions.

In another embodiment according to the invention, a method of a tandem decarboxylation and reduction of a substrate according to formula **IIIa, IIIb, IIIc, IIId,** or **IIIe** is described, wherein the method comprises bringing a substrate into contact with a catalyst according to any of the previous embodiments comprising metal nanoparticles, and heating under pressure in the presence of hydrogen 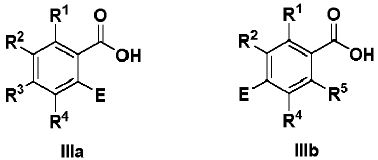 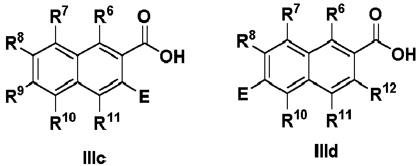 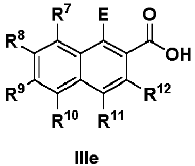 wherein:
E is -OH, -NO₂, -NH₂ or -NR¹³₂, wherein R¹³ is an alkyl group with 1-4 carbon atoms, preferably R¹³ is 1;
   preferably wherein E is -OH, -NO₂, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
   R¹⁶ is an alkyl group with 1-30 carbon atoms, preferably 1-10 carbon atoms;
   the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
   the linear or branched alkyl group contains at least one group selected from C-C double bond, C-C triple bond, keto group, ester group, ether group or amido group;
   preferably R¹ to R¹² are each independently -H, -OMe, -Me, -F, -NH₂, -OH, -NO₂, -COCH₃, -CHO, or - CO- (CH₂)₆-CH₃.

In a preferred embodiment, the substrate is a substrate according to formula **IIIa** or **IIIb**, and wherein:
E is -OH, -NO₂ or -NH₂; and
R¹ and R⁴ are each independently -H, -OMe, -Me, -F, -NH₂, -OH, -NO₂, -COCH₃, -CHO, or -CO-(CH₂)₆-CH₃.

In any of the above-described methods, the substrate can be heated at 80-250 °C. Preferably the substrate is heated at 125-200 °C, more preferably 150-200 °C.

In any of the above-described methods, the pressure can be 10-100 bar. Preferably the pressure is 20-60 bar, most preferably 40-60 bar.

In any of the above-described methods, a non-polar solvent, a polar aprotic solvent, or a polar protic solvent can be used. For metal-oxide based catalysts prepared by silanization, it is preferable not to use water. Preferably, the solvent is heptane, decalin, dioxane or mesitylene.

In any of the above-described methods involving a tandem reduction and decarboxylation reaction, the reduction is preferably a hydrodeoxygenation.

The above-described tandem reduction and decarboxylation reactions, provide a selective reduction of e.g., carbonyl and nitro functionalities, without aromatic ring hydrogenation.

### The invention includes:

**1.** A catalyst comprising:

   Su - L - Sp - C¹ - R - D

   wherein:
   Su is a support;
   L is a linker group;
   Sp is a spacer group;
   C¹ is a cation;
   R is an alkylene group with 1-20 carbon atoms,
   optionally containing one or more groups selected from ether, ester, amido or arylene groups; and
   D is a basic group,
   wherein the catalyst further comprises a counterion A¹, which is an anion.
**2.** A catalyst comprising:
   (i) the following structure **I**:

      Su - L - Sp - C¹ - R - R'

      wherein:
      Su is a support;
      L is a linker group;
      Sp is a spacer group;
      C¹ is a cation;
      R is an alkylene group with 1-20 carbon atoms,
      optionally containing one or more groups selected from ether, ester, amido or arylene groups; and
      R' is -CH₃ or D, wherein D is a basic group;
      wherein structure **I** further comprises a counterion A¹, which is an anion;
      and
   (ii) the following structure **II**:

      CH₃ - R^{a} - C² - R^{b} - D

      wherein R^{a} and R^{b} are each independently an alkylene group with 1-20 carbon atoms, optionally containing one or more groups selected from ether, ester, amido or arylene groups; C² is a cation; and D is a basic group;
      wherein structure **II** further comprises a counterion A², which is an anion.
**3.** Catalyst according to clause 1 or 2, further comprising bimetallic nanoparticles that are immobilised on the support Su, wherein the bimetallic nanoparticles are represented by:

   M¹ₓM²₁₀₀₋ₓ

   wherein:
   M¹ is a 3d transition metal;
   M² is a noble metal; and
   x is a number between 1 and 99, preferably between 10 and 80.
**4.** Catalyst according to any preceding clause, wherein R is an alkylene group with 1-10 carbon atoms, more preferably 1-6 carbon atoms, most preferably R is an alkylene group with 2-4 carbon atoms.
**5.** Catalyst according to any preceding clause, wherein R^{a} and R^{b} are each independently an alkylene group with 1-10 carbon atoms, more preferably 1-6 carbon atoms, most preferably R is an alkylene group with 2-4 carbon atoms.
**6.** Catalyst according to any preceding clause, wherein the spacer Sp is an alkylene group with 1-30 carbon atoms, optionally containing one or more groups selected from ether, ester, amido or arylene groups;
   preferably the spacer Sp is an alkylene group with 2-10 carbon atoms, most preferably with 2-4 carbon atoms.
**7.** Catalyst according to any preceding clause, wherein the support Su is selected from SiO₂, Al₂O₃, TiO₂, ZrO₂, CaO₂, ZnO₂, MgO₂, CeO₂, graphene, graphitic material, activated charcoal or carbon nanotubes; preferably wherein the support is SiO₂.
**8**. Catalyst according to any preceding clause, wherein the linker L is an -Si-O- group, a C-C linkage or an amido group connected to the support; preferably, the linker is a -Si-O-group connected to the support.
**9.** Catalyst according to any preceding clause, wherein the linker is an -Si-O- group and the spacer is an alkylene group with 1-30 carbon atoms.
**10.** Catalyst according to any preceding clause, wherein C¹ and C² are each independently selected from imidazolium, phosphonium, pyridinium, ammonium, or triazolium;
   preferably C¹ and C² are the same, more preferably C¹ and C² are imidazolium.
**11.** Catalyst according to any preceding clause, wherein A¹ and A² are each independently selected from NTf₂⁻, OTf⁻, CTf₃⁻, halides, OAc⁻, HCO₂⁻, HCO₃⁻, CO₃²⁻, NO₃⁻ BF₄ PF₆⁻ CH₃SO₃⁻, CF₃SO₃⁻, perfluorobutanesulfonate,
   perfluorooctanoate, bis[(trifluoromethyl)sulfonyl]imide, bis[(pentafluoroethyl)sulfonyl]imide, tris(pentafluoroethyl)trifluorophosphate,
   imidazolate, dimethylphosphate, or diethylphosphate;
      preferably A¹ and A² are the same, more preferably A¹ and A² are NTf₂⁻.
**12.** Catalyst according to any preceding clause, wherein the basic group D is an amine;
   preferably the amine is -NR¹⁴R¹⁵, wherein R¹⁴ and R¹⁵ are each independently H, methyl, ethyl, propyl, or butyl; more preferably R¹⁴ and R¹⁵ are ethyl.
**13.** Catalyst according to any one of clauses 3-12, wherein M¹ is Mn, Fe, Co, Ni or Cu and M² is Rh, Ru, Pd or Pt;
   preferably M¹ is Fe and M² is Ru
**14.** Catalyst according to any one of clauses 3-13, wherein x is a number between 20 and 60. More preferably, x is a number between 25 and 50.
**15.** Catalyst according to any one of clauses 3-14, wherein:
   when M¹ is Fe and M² is Ru, x is a number between 1 and 40, preferably a number between 10 and 25, most preferably wherein x is 25;
   when M¹ is Co and M² is Ru, x is a number between 50 and 70;
   when M¹ is Co and M² is Rh, x is a number between 30 and 80.
**16.** Use of a catalyst according to any one of clauses 1-15 in a decarboxylation reaction.
**17.** Use of a catalyst according to any one of clauses 3-15 in a tandem reduction and decarboxylation reaction.
**18.** Use of a catalyst according to clause 17, wherein the reduction reaction is a hydrodeoxygenation reaction.
**19.** Method of decarboxylating a substrate according to formula **IIIa, IIIb, IIIc, IIId,** or **IIIe,** wherein the method comprises bringing the substrate into contact with a catalyst comprising an ionic liquid with a basic group, and heating under pressure in the presence of hydrogen 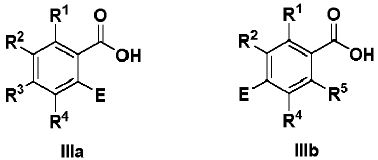 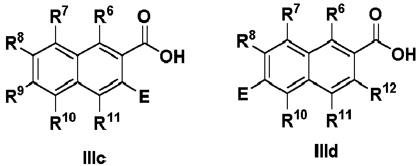 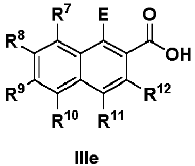 wherein:
   E is -OH, -NH₂ or -NR¹³₂, wherein R¹³ is an alkyl group with 1-6 carbon atoms, preferably R¹³ is 1;
      preferably wherein E is -OH, or -NH₂;
   R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
      R¹⁶ is an alkyl group with 1-30 carbon atoms, preferably 1-10 carbon atoms;
      the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
      the linear or branched alkyl group contains at least one group selected from C-C double bond, C-C triple bond, keto group, ester group, ether group or amido group;
      preferably R¹ to R¹² are each independently -H, -OMe, -Me, -F, -NH₂, -OH, -NO₂, -COCH₃, -CHO, or - CO-(CH₂)₆-CH₃.
20. Method of decarboxylating a substrate according to formula **IIIa, IIIb, IIIc, IIId,** or **IIIe,** wherein the method comprises bringing the substrate into contact with a catalyst comprising bimetallic nanoparticles and an ionic liquid with a basic group, which is optionally a group D as defined in other clauses, and heating under pressure in the presence of hydrogen 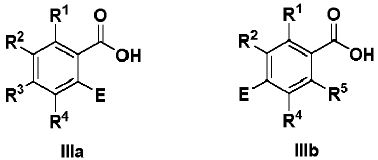 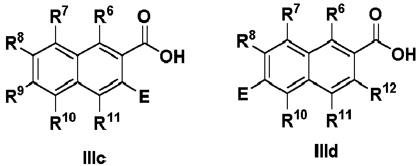 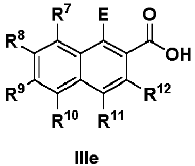 wherein:
   E is -OH, -NH₂ or -NR¹³₂, wherein R¹³ is an alkyl group with 1-6 carbon atoms, preferably R¹³ is 1;
      preferably wherein E is -OH, or -NH₂;
   R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
      R¹⁶ is an alkyl group with 1-30 carbon atoms, preferably 1-10 carbon atoms;
      the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
      the linear or branched alkyl group contains at least one group selected from C-C double bond, C-C triple bond, keto group, ester group, ether group or amido group;
      preferably R¹ to R¹² are each independently -H, -OMe, -Me, -F, -NH₂, -OH, -NO₂, -COCH₃, -CHO, or - CO- (CH₂)₆-CH₃.
**21.** Method of decarboxylating a substrate according to formula **IIIa, IIIb, IIIc, IIId,** or **IIIe,** wherein the method comprises bringing the substrate into contact with a catalyst according to any one of claims 1-15, and heating under pressure in the presence of hydrogen 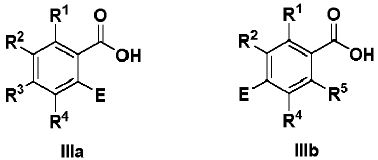 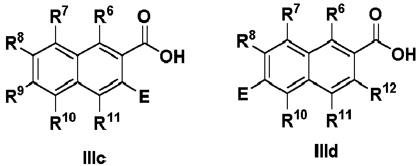 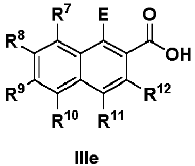 wherein:
   E is -OH, -NH₂ or -NR¹³₂, wherein R¹³ is an alkyl group with 1-6 carbon atoms, preferably R¹³ is 1;
      preferably wherein E is -OH, or -NH₂;
   R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
      R¹⁶ is an alkyl group with 1-30 carbon atoms, preferably 1-10 carbon atoms;
      the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
      the linear or branched alkyl group contains at least one group selected from C-C double bond, C-C triple bond, keto group, ester group, ether group or amido group;
      preferably R¹ to R¹² are each independently -H, -OMe, -Me, -F, -NH₂, -OH, -NO₂, -COCH₃, -CHO, or - CO-(CH₂)₆-CH₃.
**22.** Method of according to any one of clauses 19-21, wherein the substrate is a substrate according to formula **IIIa** or **IIIb**, wherein:
   E is -OH or -NH₂; and
   R¹ to R⁴ are each independently -H, -OMe, -Me, -F, -NH₂, -OH, -NO₂, -COCH₃, -CHO, or -CO-(CH₂)₆-CH₃.
**23.** Method of tandem decarboxylation and reduction of a substrate according to formula **IIIa, IIIb, IIIc, IIId,** or **IIIe**, wherein the method comprises bringing the substrate into contact with a catalyst comprising metal nanoparticles and an ionic liquid with a basic group, which is optionally a group D as defined in other clauses, and heating under pressure in the presence of hydrogen 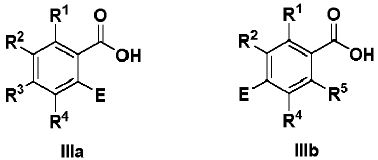 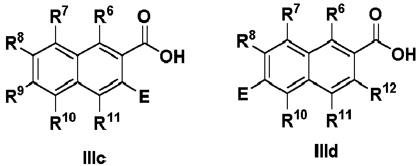 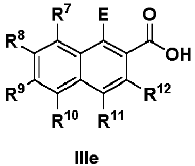 wherein:
   E is -OH, -NO₂, -NH₂ or -NR¹³₂, wherein R¹³ is an alkyl group with 1-4 carbon atoms, preferably R¹³ is 1;
      preferably wherein E is -OH, -NO₂, or -NH₂;
   R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
   R¹⁶ is an alkyl group with 1-30 carbon atoms, preferably 1-10 carbon atoms;
   the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
   the linear or branched alkyl group contains at least one group selected from C-C double bond, C-C triple bond, keto group, ester group, ether group or amido group;
   preferably R¹ to R¹² are each independently -H, -OMe, -Me, -F, -NH₂, -OH, -NO₂, -COCH₃, -CHO, or - CO-(CH₂)₆-CH₃.
**24.** Method according to clause 23, wherein the metal nanoparticles are bimetallic nanoparticles.
**25.** Method of tandem decarboxylation and reduction of a substrate according to formula **IIIa, IIIb, IIIc, IIId,** or **IIIe**, wherein the method comprises bringing the substrate into contact with a catalyst according to any one of clauses 3-15, and heating under pressure in the presence of hydrogen 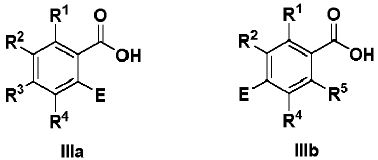 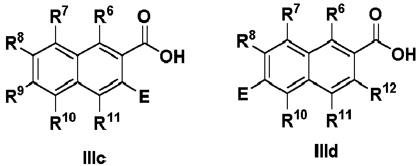 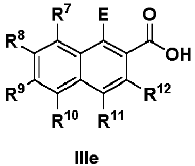 wherein:
   E is -OH, -NO₂, -NH₂ or -NR¹³₂, wherein R¹³ is an alkyl group with 1-4 carbon atoms, preferably R¹³ is 1;
      preferably wherein E is -OH, -NO₂, or -NH₂;
   R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
      R¹⁶ is an alkyl group with 1-30 carbon atoms, preferably 1-10 carbon atoms;
      the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
      the linear or branched alkyl group contains at least one group selected from C-C double bond, C-C triple bond, keto group, ester group, ether group or amido group;
      preferably R¹ to R¹² are each independently -H, -OMe, -Me, -F, -NH₂, -OH, -NO₂, -COCH₃, -CHO, or - CO-(CH₂)₆-CH₃.
**26.** Method according to clause 23, 24 or 25, wherein the substrate is a substrate according to formula **IIIa** or **IIIb**, and wherein:
   E is -OH, -NO₂ or -NH₂; and
   R¹ and R⁴ are each independently -H, -OMe, -Me, -F, -NH₂, -OH, -NO₂, -COCH₃, -CHO, or -CO-(CH₂)₆-CH₃.
**27.** Method according to any one of clauses 19-26, wherein the substrate is heated at 80-250 °C, preferably 125-200 °C, more preferably 150-200 °C.
**28.** Method according to any one of clauses 19-27, wherein the pressure is 10-100 bar, preferably 20-60 bar, most preferably 40-60 bar.
**29.** Method according to any one of clauses 19-28, wherein a solvent is present.
**30.** Method according to clause 29, wherein the solvent is a non-polar solvent, a polar aprotic solvent or a polar protic solvent.
**31.** Method according to clause 29, wherein the solvent is heptane, decalin, dioxane or mesitylene.
**32.** Method according to clause 30, wherein when the catalyst is metal-oxide based, water is not used as a solvent.
**33.** Method according to any one of clauses 23-32, wherein the reduction reaction is a hydrodeoxygenation reaction.
**34.** Method of tandem decarboxylation and reduction of a substrate according to formula **IIIa** or **IIIb**, 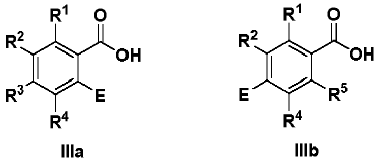 wherein E is -OH, -NO₂ or -NH₂; and R¹ and R⁴ are each independently -H, -OMe, -Me, -F, -NH₂, -OH, -NO₂, -COCH₃, -CHO, or -CO-(CH₂)₆-CH₃;
   wherein the method comprises bringing the substrate into contact with a catalyst comprising:
   (i) the following structure **I**:

      Su - L - Sp - C¹ - R - CH₃

      wherein:
      Su is a support, which is SiO₂;
      L is a linker group, which is a -Si-O- group connected to the support;
      Sp is a spacer group, which is an alkylene group with 2-4 carbon atoms;
      C¹ is imidazolium; and
      R is an alkylene group with 3 carbon atoms;
      wherein structure **I** further comprises a counterion A¹, which is NTf₂⁻;
   (ii) the following structure **II**:

      CH₃ - R^{a} - C² - R^{b} - NEt₂

      wherein R^{a} is an alkylene group with 3 carbon atoms and R^{b} is an alkylene group with 4 carbon atoms; and C² is imidazolium;
      wherein structure **II** further comprises a counterion A², which is NTf₂⁻; and
   (iii) bimetallic nanoparticles that are immobilised on the support Su, wherein the bimetallic nanoparticles are represented by:

      M¹ₓM²₁₀₀₋ₓ

      wherein M¹ is a Fe or Co, preferably Fe; M² is Ru or Rh, preferably Ru; and x is a number between 10 and 80, preferably between 10 and 30.
   and heating under pressure in the presence of hydrogen.

### Examples

Examples according to the present invention will be presented.

### General:

Unless stated otherwise, the synthesis of ionic liquids (ILs), supported ionic liquid phases (SILPs) and nanoparticles immobilised on SILPs (NPs-SILPs) were carried out under an inert atmosphere (Ar) using standard Schlenk techniques or inside a glovebox. All synthesised materials were stored under inert atmosphere.

### Autoclave reactions:

As an example, the autoclave reaction procedure for the decarboxylation of 4-hydroxybenzoic acid is described. For other substrates, the amounts of substrate, catalyst or solvent and the reaction conditions can be varied. However, the overall procedure stays the same.

The substrate (65 eq. with respect to metal, 0.221 mmol), catalyst (10 mg, containing 0.0034 mmol metal) and solvent (heptane, 0.5 mL) were weighed inside of a glovebox into the autoclave. The autoclave was closed, transferred out of the glovebox and pressurized with 50 bar hydrogen. After heating the autoclave at 175 °C under stirring (500 rpm) for 18 h, it was cooled down in a water bath and depressurized. The GC samples were prepared by adding acetone (250 mg) to the reaction mixture and taking a sample through a syringe with a filter. To the sample, tetradecane (internal standard, 20 mg) and acetone (200 mg) were added. For all amines, methanol/dioxane (1/1) was used as solvent for the GC samples.

### Isolation of Products:

The isolation of the products was carried out by filtration of the reaction mixture through a syringe filter and purification *via* silica column (EtOAc:Cyclohexane = 1:10). After careful evaporation of the solvent, the pure compounds were yielded and characterized by ¹H and ¹³C NMR.

### Analytical purposes:

All solution state NMR were recorded on a Bruker Ascend 400 spectrometer at room temperature. The coupling constants (*J*) are given in Hertz (*H*z), and the chemical shifts (δ) are expressed in ppm, relative to TMS at 25 °C. Gas chromatography (GC) was performed on a Shimadzu GC-2030 equipped with a FID-detector and a CP-WAX-52CB column from Agilent. Gas chromatography coupled with a mass spectrometer (GC-MS) were performed on a Shimadzu QP2020. N₂ adsorption experiments were performed on a Quadrasorb SI from Quantachrom Instruments. Transmission and scanning transmission electron microscopy (TEM and STEM) were performed on a Hitachi HF2000 cold FEG operating at 200 kV at the Max-Planck-Institut für Kohlenforschung. Samples were prepared by depositing the powder onto a copper TEM grid with an amorphous carbon support film. Scanning electron microscopy with energy dispersive X-ray spectroscopy (SEM/EDS) was performed on a Hitachi S-3500N operating at 30kV at the Max-Planck-Institut für Kohlenforschung. Samples were prepared by depositing the powder onto a tape and flattening it out to obtain an even surface for analysis.

### GC-Analysis:

Yields were determined by gas chromatography (Shimadzu Nexis 2030) equipped with a FID detector using the methods described in the table below. Method 1 was used for substrate 1 and 17. Method 2 was used for substrate 9. Method 3 was used for substrates 6 and 12. Method 4 was used for substrate 16 and method 5 was used for substrates 1-5, 7, 8, 10, 11, 13-15.

The identification of the compounds was done by injecting the pure compounds in GC-FID, or by combination with GC-MS.

**Table 1: Methods for GC analysis**

| **Method Nr.** | **1** | **2** | **3** | **4** | **5** |
|---|---|---|---|---|---|
| **Stationary phase (Column)** | Rtx-1701 (0.25 µm, 0.25 mm, 30 m) | Rtx-1 (0.5 µm, 0.25 mm, 30 m) | Rtx-5 (1.0 µm, 0.25 mm, 30 m) | Rtx-1 (0.5 µm 0.25 mm, 30 m) | Rtx-1 (0.5 µm, 0.25 mm, 30 m) |
| **Mobile Phase [mL/min] (Carrier Gas)** | 1.9 (He) | 1.22 (He) | 1.71 (He) | 1.25 (He) | 1.21 (He) |
| **Flow Control Mode Linear Velocity [cm/sec]** | 40 | 30 | 40 | 30 | 30 |
| **Injection Volume [µl]** | 0.5 | 0.2 | 0.6 | 0.6 | 0.2 |
| **Injection Temperature [°C]** | 270 | 250 | 320 | 250 | 250 |
| **Split Ratio** | 25 | 30 | 30 | 35 | 30 |
| **Temperature Program [°C]** | 50 for 5 min, to 270 with 20 °C/min, 270 for 9 min | 60 for 12 min, to 200 with 12 °C/min, 200 for 8 min | 100 for 1 min, to 320 with 10 °C/min, 320 for 2 min | 50 for 8 min, to 200 with 15 °C/min, to 300 with 25 °C/min, 300 for 22 min | 65 for 10 min, to 200 with 15 °C/min, 200 for 8 min |
| **Detector Temperature [°C]** | 275 | 260 | 330 | 310 | 260 |

### Example 1: Method of preparing example catalyst comprising i) SILP^{A}, ii) ionic liquid with basic group and iii) bimetallic nanoparticles Fe₂₅Ru₇₅

SILP^{A} is composed of [1-butyl-3-(3-triethoxysilylpropyl)imidazolium] chemisorbed on SiO₂.

The new multifunctional catalyst (Fe₂₅Ru₇₅-SILP**^{A}** + IL-NEt₂) was assembled in a versatile manner through physisorption of the basic ionic liquid 1-[2-(diethylamino)ethyl]-3-butylimidazolium bis(trifluoromethane)sulfonamide (IL-NEt₂) on Fe₂₅Ru₇₅-SILP^{A} material. In brief, the preparation of Fe₂₅Ru₇₅-SILP^{A} involved the condensation of [1-butyl-3-(3-triethoxy-silylpropyl)-imidazolium]NTf₂ with dehydroxylated SiO₂, followed by *in situ* reduction of a mesitylene solution of {Fe[N(Si(CH₃)₃₎₂]₂}₂ and [Ru(cod) (cot)] (cod = cyclooctadiene; cot = cyclooctatriene) under an atmosphere of H₂ (3 bar) at 150 °C (for detailed procedure see below). The physisorption of the amine functionalized ionic liquid [1-butyl-3-(2-(diethylamino)ethyl)-imidazolium]NTf₂ (IL-NEt₂) to prepare the bifunctional catalyst (Fe₂₅Ru₇₅-SILP**^{A}** + IL-NEt₂) was achieved by stirring a suspension of Fe₂₅Ru₇₅-SILP**^{A}** and IL-NEt₂ in acetone at room temperature.

The BET surface area (36 m²·g⁻¹) and the pore volume (0.13 m³-g⁻¹) measured by N₂ adsorption experiments were significantly reduced upon physisorption of IL-NEt₂ on Fe₂ₛRu₇₅-SILP**^{A}**, indicating that the physisorption occurred:

**Table 2**

| | **Fe₂₅Ru₇₅-SILP^{A}** | **Fe₂₅Ru₇₅-SILP^{A}+IL-NEt₂** |
|---|---|---|
| **IL-NEt₂ loading [mmol·g⁻¹]** | | 0.73^{[a]} |
| **Accessible amines^{[b]} [mmol·g⁻¹]** | - | 0.48 |
| **BET surface area [m²·g⁻¹]** | 240 ± 1 | 36 ± 1 |
| **Pore volume [m³·g⁻¹]** | 0.56 ± 0.002 | 0.13 ± 0.002 |
| **Metal loading^{[b]} [mmol·g⁻¹]** | 0.40 ± 0.04 | 0.34 ± 0.03 |
| **Metal ratio Fe** : **Ru^{[b]} [%]** | 23 ± 5 : 77 ± 5 | 27 ± 3 : 73 ± 4 |
| **Particle size [nm]** | 3.3 ± 0.6 | 3.1 ± 0.7 |

| | | |
|---|---|---|
| NP sizes were determined by TEM. Metal ratio and loading were determined using SEM-EDX (Fe-K / Ru-L). ^{[a]} Determined based on the amount of IL-NEt₂ recovered after the physisorption procedure. ^{[b]} The average values and standard deviations were determined through the characterization of three batches of each material. | | |

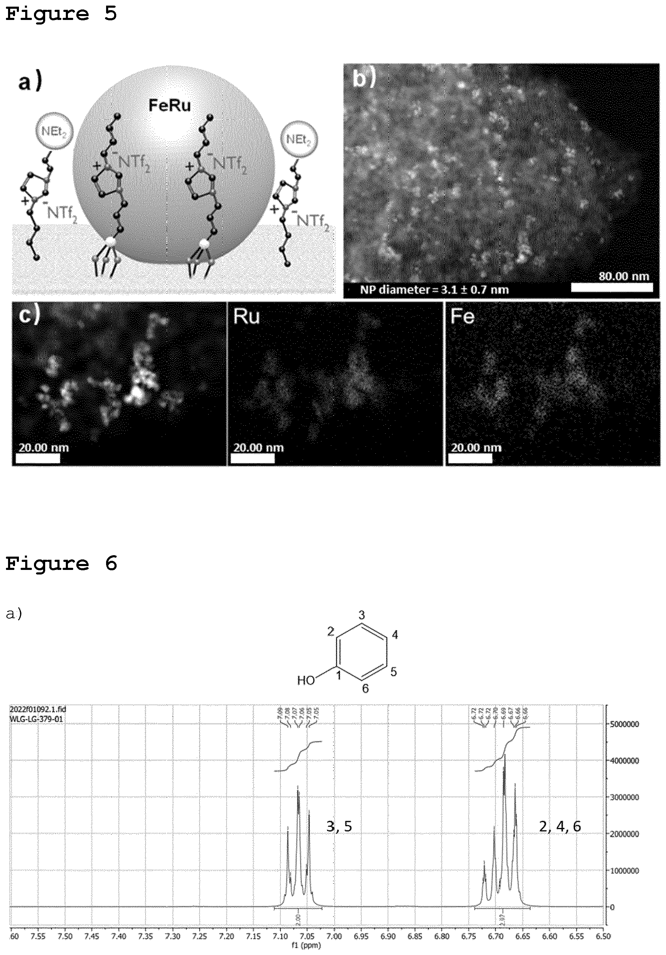

Titration of the amount of accessible amine functionalities on Fe₂₅Ru₇₅-SILP**^{A}** + IL-NEt₂ gave 0.48 mmol·g⁻¹, which corresponds to 64% of the theoretical total amount for quantitative adsorption. Characterization of Fe₂₅Ru₇₅-SILP + IL-NEt₂ by scanning transmission electron microscopy in high angle annular dark field (STEM-HAADF) associated to energy dispersive X-ray spectroscopy (EDS) showed the presence of small (3.1 nm) bimetallic NPs containing Fe and Ru (Fig. 5). Scanning electron microscopy (SEM) with EDS confirmed the expected metal ratio and metal loading (0.34 mmol·g⁻¹, Table 2). Thus, physisorption did not significantly affect the metal loading or ratio, nor the size of the metal NPs, even though they were slightly more aggregated than on the pristine Fe₂₅Ru₇₅-SILP^{A} material.

An example synthesis of an ionic liquid is provided as follows:

### Step A: Synthesis of 1-[2-(diethylamino)ethyl]-3-butylimidazolium bromide hydrobromide

2-Bromo-N,N-diethylethylamine hydrobromide (7.26 g, 27.8 mmol) was suspended in 100 mL dry acetonitrile and heated at 80 °C until complete dissolution, giving a light yellow solution. Under Ar, 1-butylimidazole (3.6 mL, 29 mmol) was added slowly to the mixture under stirring and the reaction medium was refluxed at 150 °C for 48 h. After this period, the reaction was left to cool down to room temperature, the solvent was evaporated and the resulting yellow paste was washed with dry acetone (4 x 10 mL) and dry diethylether (4 x 10 mL). The remaining solid was dried under vacuum for 3 h at room temperature and 1-[2-(diethylamino)ethyl]-3-butylimidazolium bromide hydrobromide was yielded as white solid (6.27 g, 20.60 mmol, 74%).

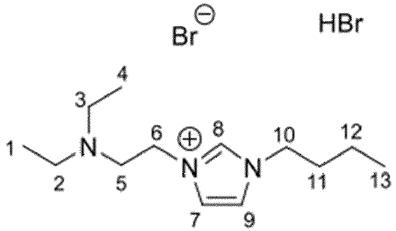

¹H-NMR (400 MHz, DMSO-*d*₆) : δ (ppm) = 9.80 (s, 1H, HBr), 9.41 (s, 1H, H8), 7.95 (s, 1H, H9), 7.86 (s, 1H, H7), 4.66 (t, *J* = 6.8 Hz, 2H, H6), 4.19 (t, *J* = 7.2 Hz, 2H, H10), 3.66 (t, *J* = 7.0 Hz, 2H, H5), 3.23 (q, *J* = 7.3 Hz, 4H, H2, H3), 1.84-1.77 (m, 2H, H11), 1.35-1.23 (m, 8H, H1, H4, H12), 0.92 (t, *J* = 7.3 Hz, 3H, H13).

¹³C-NMR (400 MHz, DMSO-*d6*) : δ (ppm) = 137.42 (1C, C8), 123.13 (1C, C9), 123.06 (1C, C7), 49.88 (1C, C5), 49.21 (1C, C6), 47.43 (2C, C2, C3), 43.61 (1C, C10), 31.34 (1C, C11), 19.29 (1C, C12), 13.80 (1C, C13), 8.86 (2C, C1, C4).

### Step B: Synthesis of 1-[2-(diethylamino)ethyl]-3-butylimidazolium bis(trifluoromethane) sulfonamide

1-[2-(diethylamino)ethyl]-3-butylimidazolium bromide hydrobromide (10.94 g, 28.41 mmol) was dissolved in ethanol (60 mL) under an inert atmosphere. Under Ar, pyridine (2.4 mL, 2.41 g, 30.51 mmol, 1.07 equiv.) was added to the mixture, along with additional 10 mL of ethanol to rinse the Schlenk flask, after which the reaction medium was stirred for 10 min at room temperature. Finally, LiNTf₂ (8.64 g, 30.09 mmol) was added to the solution, along with additional 10 mL of ethanol to rinse, and the resulting solution was stirred under Ar at room temperature for 15 h. Afterwards, the solvent was evaporated and the resulting pale orange residue was dissolved in DCM (40 mL). The solution was filtered through celite (previously washed with 2 x 10 mL DCM). Additional 20 mL of DCM were added to rinse the flask. Due to the remaining of a small amount of a whitish thin solid in suspension, the solution was filtered through celite a second time, giving a clear light-yellow solution. Finally, the solvent was removed from the recovered solution. The product was dissolved and stirred for 90 min in a mixture of H₂O (30 mL) and DCM (60 mL) to remove any remaining trace of pyridine. The organic layer was washed with 3 x 20 mL H₂O, dried with anhydrous Na₂SO₄ and finally the solvent was removed under vacuum to afford a thick light-yellow oil that was dried under vacuum overnight at room temperature. 1-[2-(diethylamino)ethyl]-3-butylimidazolium bis(trifluoromethane) sulphonamide was obtained as a viscous liquid (7.95 g, 15.76 mmol, 77%).

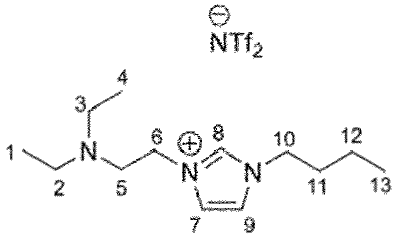

¹H-NMR (400 MHz, DMSO-*d6*) : δ (ppm) = 9.17 (s, 1H, H8), 7.80 (d, J = 8.9 Hz, 1H, H7, H9), 4.43 (t, J = 6.4 Hz, 2H, H6), 4.20 (t, *J* = 7.2 Hz, 2H, H10), 3.26 (t, *J* = 6.1 Hz, 2H, H5), 2.92 (q, 4H, H2, H3), 1.83-1.75 (m, 2H, H11), 1.34-1.24 (m, 2H, H12), 1.08 (t, *J* = 7.2 Hz, 6H, H1, H4), 0.92 (t, *J* = 7.4 Hz, 3H, H13).

¹³C-NMR (400 MHz, DMSO-*d6*) : δ (ppm) = 136.68 (1C, C8), 122.79 (1C, C9), 122.41 (1C, C7), 50.41 (1C, C5), 48.72 (1C, C6), 46.82 (2C, C2, C3), 44.99 (1C, C10), 31.40 (1C, C11), 18.78 (1C, C12), 13.17 (1C, C13), 9.70 (2C, C1, C4).

APCI-MS (+) : m/z = 224.2, calculated for [C₁₃H₂₆N₃]⁺ = 224.21. APCI-MS (-): m/z = 279.8, calculated for [C₂F₆NO₄S₂]⁻ = 279.92; m/z = 566.9, calculated for [LiNTf₂+NTf₂]⁻, [C₄F₁₂LiN₂O₈S₄]⁻ = 566.85.

### Detailed synthesis of the Fe₂₅Ru₇₅-SILP^{A} + IL-NEt₂ catalyst:

### 1.1. Synthesis of SILP^{A}

[1-Butyl-3-(3-triethoxysilylpropyl)imidazolium]NTf₂ (5.49 g, 8.80 mmol) was dissolved in DCM (20 mL) and added to a suspension of dehydroxylated SiO₂ (10.0 g in 50.0 mL toluene). After stirring for 15 min at room temperature (rt), DCM was removed in vacuo and the resulting mixture was refluxed for 18 h. Upon removal of the organic phase, the SILP**^{A}** was washed with DCM (3x25 mL) and dried in vacuo.

### 1.2. Synthesis of Fe₂₅Ru₇₅-SILP^{A}

A solution of {Fe[N(Si(CH₃)₃)₂]₂}₂ (18.0 mg, 0.05 mmol Fe) in mesitylene (2 mL) was combined with a solution of [Ru(cod) (cot)] (47.0 mg, 0.15 mmol Ru) in mesitylene (2 mL) in a Fischer-Porter bottle (70 mL). The SILP (500 mg) was added to the solution of the metal precursors along with mesitylene (1 mL) and the reaction mixture was stirred under argon at room temperature for 30 min. The Fischer-Porter bottle was evacuated and backfilled with H₂ and the suspension was stirred under H₂ (3 bar) at 150 °C for 18 h. Under this reducing environment a black powder was obtained, indicating the immobilization of the NPs onto the SILP. Mesitylene was decanted and Fe₂₅u₇₅-SILP were washed with fresh toluene (3 x 3 mL) and dried *in vacuo* at room temperature for 1 h.

### 1.3. Synthesis of Fe₂₅Ru₇₅-SILP^{A} + IL-NEt₂.

The physisorption of IL-NEt₂ onto Fe₂₅Ru₇₅-SILP**^{A}** was achieved through the following: A suspension of Fe₂₅Ru₇₅-SILP**^{A}** (187.9 mg, 0.0742 mmol) was prepared in an acetone solution (3 mL) of IL-NEt₂ (109.6 mg, 0.217 mmol, 2.9 equiv.). The mixture, prepared in a glove box, was stirred for 1 h at room temperature, after which it was taken out of the glove box and the solvent was evaporated in a Schlenk line. Finally, the catalyst was dried under vacuum for 2 h at room temperature.

The above-described synthetic methodology can be applied in the synthesis of other catalysts of the general formula M₁M₂-SILP + IL-D, by exchanging the relevant components.

### Example 2: Catalytic performance of the bifunctional catalyst Fe₂₅Ru₇₅-SILP^{A} + IL-NEt₂ and further reference materials in the decarboxylation of 4-hydroxybenzoic acid (1) as model reaction

The standard conditions were set to 175°C, 18 h, heptane as solvent, and 65 and 30 equivalents of substrate with respect to the total metal loading and the amine groups of IL-NEt₂, respectively.

**Table 3: Decarboxylation of 4-hydroxybenzoic acid using Fe₂₅Ru₇₅-SILP^{A} + IL-NEt₂ and various reference materials**

| 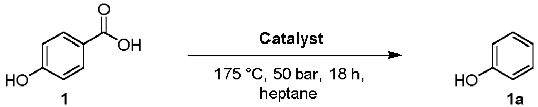 | | | | |
|---|---|---|---|---|
| **#** | **Catalyst** | **Applied gas** | **X [%]** | **Y₁ₐ [%]** |
| 1 | Fe₂₅Ru₇₅-SILP^{A}+IL-NEt₂ | - | - | - |
| 2 | Fe₂₅Ru₇₅-SILP^{A}+IL-NEt₂ | N₂ | 18 ± 5 | 18 ± 5 |
| 3 | Fe₂₅Ru₇₅-SIL^{A} +IL-NEt₂ | Ar | 10 | 10 |
| 4 | Fe₂₅Ru₇₅-SIL^{A} +IL-NEt₂ | H₂ | >99 | >99 |
| 5 | - | H₂ | 0 | 0 |
| 6 | Fe₂₅Ru₇₅-SILP^{A} | H₂ | 4 ± 1 | 4 ± 1 |
| 7 | SILP+IL-NEt₂ | H₂ | 58 ± 2 | 58 ± 2 |
| 8 | Fe₄₀Ru₆₀-SILP^{A} +IL-NEt₂ | H₂ | 33 ± 2 | 33 ± 2 |
| 9 | Fe₆₀Ru₄₀-SILP^{A} +IL-NEt₂ | H₂ | 37 ± 1 | 37 ± 1 |
| 10 | Fe₁₀₀-SiLP^{A} | H₂ | 0 | 0 |
| 11 | RU₁₀₀-SILP^{A} | H₂ | >99 | 0^{[b]} |
| 12 | Fe₂₅Ru₇₅-SiO₂ | H₂ | >99 | 39^{[c]} |
| 13 | Fe₂₅Ru₇₅-SILP^{A} +IL-NEt₂ | D₂^{[a]} | >99 | >99 |
| 14 | Fe₂₅Ru₇₅-SILP^{A} + additive: Et₃N^{[d]} | H₂ | >99 | >99 |
| 15 | Fe₂₅Ru₇₅-SILP^{A} + additive: DMA^{[e]} | H₂ | >99 | >99 |
| 16 | Fe₂₅Ru₇₅-SILP^{A} + additive: TPA^{[f]} | H₂ | 18 | 18 |
| 17 | Fe₂₅Ru₇₅-SILP^{A}+IL-SO₃H | H₂ | 0 | 0 |

| | | | | |
|---|---|---|---|---|
| Standard reaction conditions: Catalyst (10 mg, metal content: 0.0034 mmol), substrate (0.221 mmol, 65 eq. compared to metal), solvent: heptane (0.5 mL), 175°C, H₂ (50 bar), 18 h, 500 rpm; X: Conversion, Y: Yield, determined by GD-FID using tetradecane as internal standard. Variations are shown for two independent experiments. ^{[a]} 20 bar D₂, 200 °C. ^{[b]} product = cyclohexanol (99%). ^{[c]} byproducts = cyclohexanol (38%) and cyclohexane (23%). ^{[d]} Et₃N=triethylamine, Substrate/Amine = 19-0.1 equivalents. ^{[e]} DMA = dimethylaniline, Substrate/Amine = 2.5-0.7 equivalents. ^{[f]} TPA = triphenylamino, Substrate/Amine = 1 equivalent. | | | | |

Performing this reaction without applying any pressure resulted in extremely low mass balance (Table 3, Entry 1). Using 50 bar of inert gas pressure (N₂ or Ar) allowed observing significant conversion of **1** to phenol (**1a**), however still with low yields (18% and 10%, respectively) and unclosed mass balances (Table 3, Entries 2-3). In contrast, **1** was fully and selectively decarboxylated using Fe₂₅Ru₇₅-SILP^{A} + IL-NEt₂ under an H₂ atmosphere (50 bar), affording **1a** in quantitative yield without reduction of the aromatic moiety (Table 3, Entry 4).

Without catalyst, no conversion was observed under these conditions (Table 3 Entry 5). Similarly, using the bimetallic Fe₂₅Ru₇₅-SILP^{A} catalyst without physisorbed IL resulted in very low decarboxylation activity (4% yield, Table 3, Entry 6). With SILP+IL-NEt₂ in the absence of Fe₂₅Ru₇₅ NPs, the conversion and yield of **1a** reached 58% (Table 3, Entry 7).

Lowering the amount of Ru in the bimetallic particles in Fe₄₀Ru₆₀-SILP^{A}+IL-NEt₂ and Fe₆₀Ru₄₀-SILP^{A}+IL-NEt₂ resulted in lower conversions and yields (Table 3, Entries 8-9). Fe₁₀₀-SILP^{A} gave no activity (Table 3, Entry 10), while Ru₁₀₀-SILP^{A} led to complete hydrogenation of the aromatic ring (Table 3, Entry 11). Bimetallic FeRu NPs prepared directly on silica (Fe₂₅Ru₇₅-SiO₂) gave very low selectivity toward the formation of **1a** (Table 3, Entry 12) accompanied by significant aromatic ring hydrogenation indicating the presence of monometallic Ru-particles. IL-modifiers have the beneficial role of helping to avoid segregated particles upon synthesis of bimetallic NPs.

### Example 3: Experiments with D₂

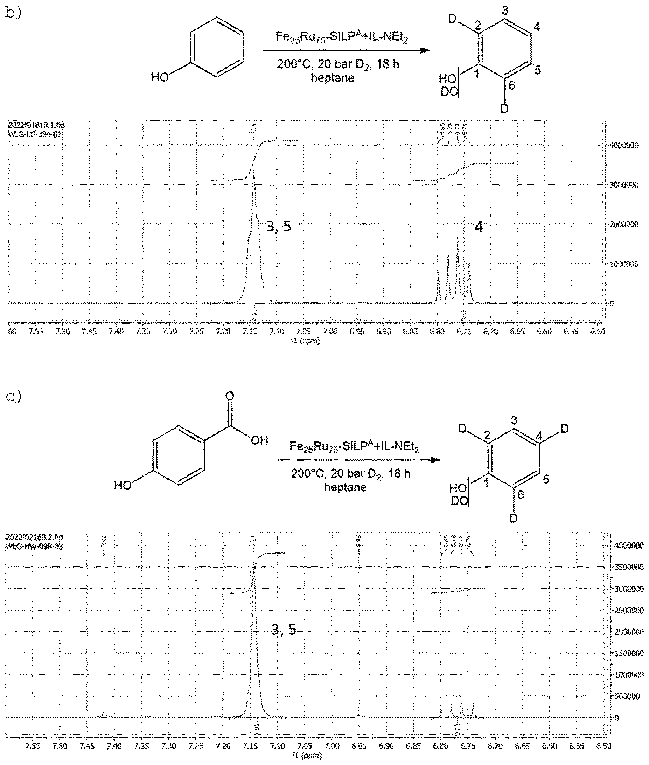

In order to get additional insight into the beneficial role of the hydrogen atmosphere in the decarboxylation reaction, the reaction was performed under standard conditions in presence of D₂ (20 bar). Full conversion to phenol was observed in this case as well (Table 3, Entry 13), and ¹H and ¹³C NMR of the isolated product revealed deuterium incorporation in *ortho* and *para* of the hydroxyl functionality (Table 4 and Figure 6), with a 78% of the product being deuterated in para.

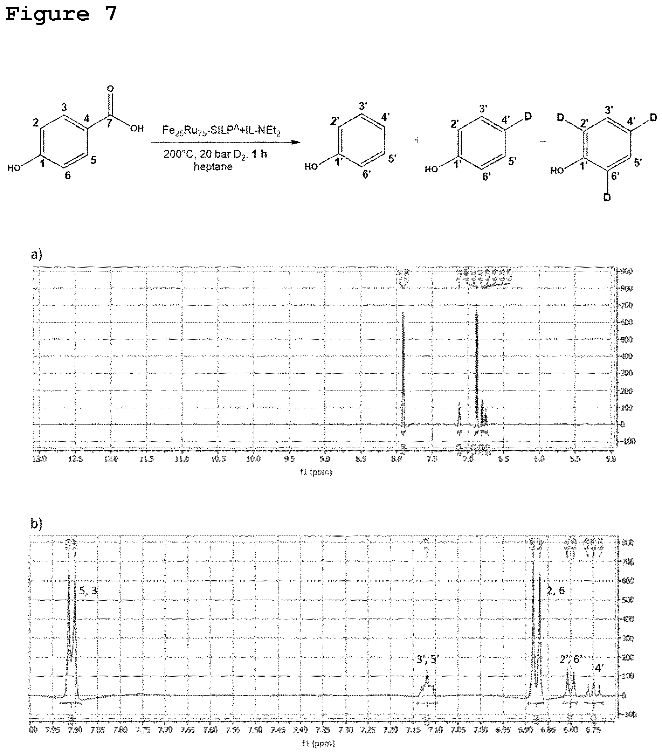

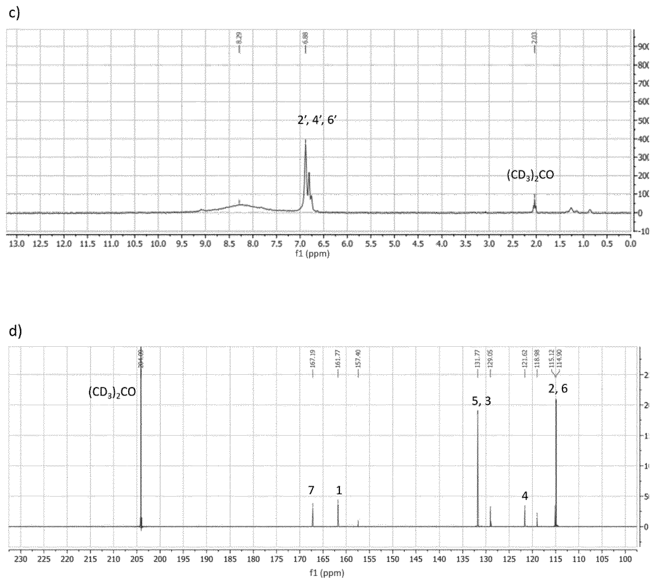

Exposing the product **1a** to D₂ under standard reaction conditions led to H/D exchange in *ortho* to the hydroxyl functionality only (Table 4 and Figure 6). This demonstrates that the deuterium incorporation in the *para* position occurs as a result of the decarboxylation process at this position. While no significant H/D exchange of the proton at the carboxylic acid functionality was observed within the first 1 h when 22% conversion was achieved, incorporation of D in the product in the *para* position was clearly detected (40% of the product, Figure 7). These results suggest that the incorporation of D in the *para* position occurs during the decarboxylation reaction, probably as a consequence of heterolytic activation of D₂ by the NPs generating D⁺ assisted by the base.

### Detailed reaction conditions:

4-Hydroxybenzoic acid (61.1 mg, 65 eq. with respect to metal, 0.442 mmol), Fe₂₅Ru₇₅-SILP^{A}+IL-NEt₂ (20 mg, containing 0.007 mmol metal) and solvent (heptane, 1.0 mL) were weighed inside of a glovebox into the autoclave. The autoclave was closed, transferred out of the glovebox and pressurised with 20 bar deuterium. After heating the autoclave under stirring (500 rpm) to 200 °C for 18 h, it was cooled down in a water bath and depressurized. The isolation of the product was carried out by purification *via* silica column (EtOAc:Cyclohexane = 1:10) . After careful evaporation of the solvent, the pure compound was yielded. The product was analyzed by GC-MS and NMR.

As a reference experiment, phenol was used as substrate under the same reaction conditions and the product was isolated and analysed accordingly.

Comparing the ¹H (Figure 6) and ¹³C NMR of the isolated products to the NMR of phenol showed a different excess of deuteration of the products (Table 4). Comparing the ¹H and ¹³C NMR of this isolated product to that of non-deuterated phenol evidenced the nearly complete deuteration of the positions 2, 4, and 6 of the aromatic ring. This deuteration caused a triplet shape of the carbon peaks of the carbons in position 2, 4 and 6 in ¹³C{¹H} NMR. In contrast, exposing non-deuterated phenol to the same reaction conditions led to a phenol with deuteration only on positions 2 and 6, as expected in alpha of the -OH group. Here, the signal for the carbon in position 4 in ¹³C{¹H} NMR is a singlet. These results show that a proton from the gas phase replaces the carboxylic acid functionality during decarboxylation and evidences the significance of having an H₂ atmosphere on decarboxylation activity. This also highlights the role of the amine functionalities and FeRu NPs, the first being responsible for the activation of the carboxylic acid functionality while the second presumably accelerates the cleavage reaction by activating H₂, thus providing protons and facilitating the protonolysis step.

**Table 4: Deuterated positions of the products isolated from the decarboxylation of 4-hydroxybenzoic acid (1) using Fe₂₅Ru₇₅-SILP^{A} + IL-NEt₂ with application of D₂**

| **Entry** | **Substrate** | **Main product** | **Deuteration Position x^{[a]} (Signal shape in ¹³C NMR)** | | | | |
|---|---|---|---|---|---|---|---|
| | | | 2 | 3 | 4 | 5 | 6 |
| 1 | 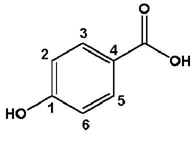 | 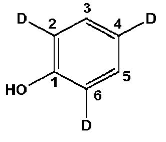 | yes (t) | no | **yes (t)** | no | yes (t) |
| 2 | 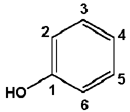 | 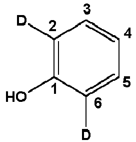 | yes (t) | no | **no (s)** | no | yes (t) |

| | | | | | | | |
|---|---|---|---|---|---|---|---|
| ^{[a]} Determined by ¹H and ¹³C{¹H} NMR of the isolated products; s = singlet, t = triplet. Reaction conditions: Catalyst (20 mg, metal content: 0.0068 mmol), substrate (0.442 mmol, 65 eq. with respect to metal), solvent: heptane (1 mL), 200 °C, D₂ (20 bar), 18 h, 500 rpm. | | | | | | | |

### Example 4: Selective decarboxylation of various aromatic carboxylic acids

Based on these promising results, the Fe₂₅Ru₇₅-SILP^{A}+IL-NEt₂ catalyst was applied to a scope of benzoic acids possessing hydroxyl, methoxy and amine substituents (Table 5). Lignin-derived 4-hydroxybenzoic acid derivatives 1-3 were readily decarboxylated, giving the corresponding phenols (1a-3a) in excellent selectivity and yields (Table 5, Entry 1-3).

Substrates 4-6 with electron donating or withdrawing groups in position 3 were also quantitatively converted to the corresponding phenols (Table 5, Entry 4-6). Similar results were obtained with salicylic acid (7), in which the hydroxyl functionality is in *ortho* position. In sharp contrast, no conversion was observed when using 3-hydroxybenzoic acid (8) as substrate, suggesting that mesomeric effects are important to observe decarboxylation activity with Fe₂₅Ru₇₅-SILP^{A}+IL-NEt₂. Notably, also anthranilic acid (9) was decarboxylated effectively to give aniline in quantitative yield (Table 3, Entry 9), showing that the decarboxylation activity of Fe₂₅Ru₇₅-SILP^{A}+IL-NEt₂ is not limited to hydroxybenzoic acid derivatives. In this case, the temperature was reduced to 150 °C to avoid side reactions (e.g. deamination). Possibility of product isolation was demonstrated for products 1a and 3a (91 and 95% isolated yield, respectively). Notably, 2,6-dimethoxyphenol (3a) is used for the synthesis of cellular anti-proliferate active agents and becomes thus accessible from lignin-derived syringic acid.

**Table 5: Decarboxylation of aromatic carboxylic acids**

| 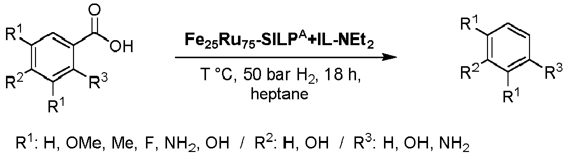 | | | | | |
|---|---|---|---|---|---|
| # | **Substrate** | **Product** | **T [°C]** | **X [%]** | **Y [%]** |
| 1 | 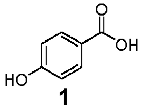 | 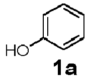 | 175 | >99 | >99 (91) |
| 2 | 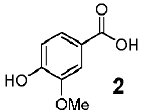 | 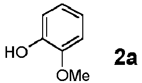 | 200 | >99 | >99 |
| 3 | 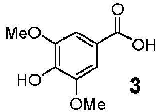 | 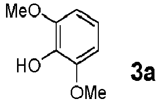 | 200 | >99 | >99 (95) |
| 4 | 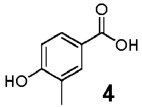 | 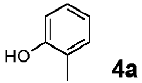 | 175 | >99 | >99 |
| 5 | 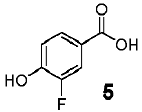 | 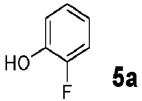 | 175 | >99 | >99 |
| 6 | 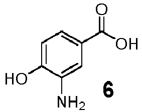 | 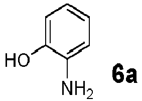 | 175 | >99 | >99 |
| 7 | 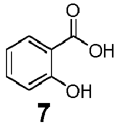 | 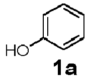 | 200 | >99 | >99 |
| 8 | 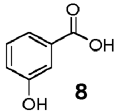 | 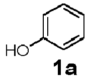 | 200 | 0 | 0 |
| 9 | 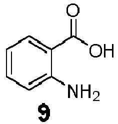 | 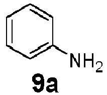 | 150 | >99 | >99 |

| | | | | | |
|---|---|---|---|---|---|
| Reaction conditions: Catalyst (10 mg, metal content: 0.0034 mmol), substrate (0.221 mmol, 65 eq. related to total metal loading), solvent: heptane (0.5 mL), H₂ (50 bar), 18 h, 500 rpm; Y: Yield, X: Conversion, determined by GD-FID using tetradecane as internal standard. Isolated yields in parenthesis. | | | | | |

### Example 5: Tandem reduction and decarboxylation of substituted aromatic carboxylic acids

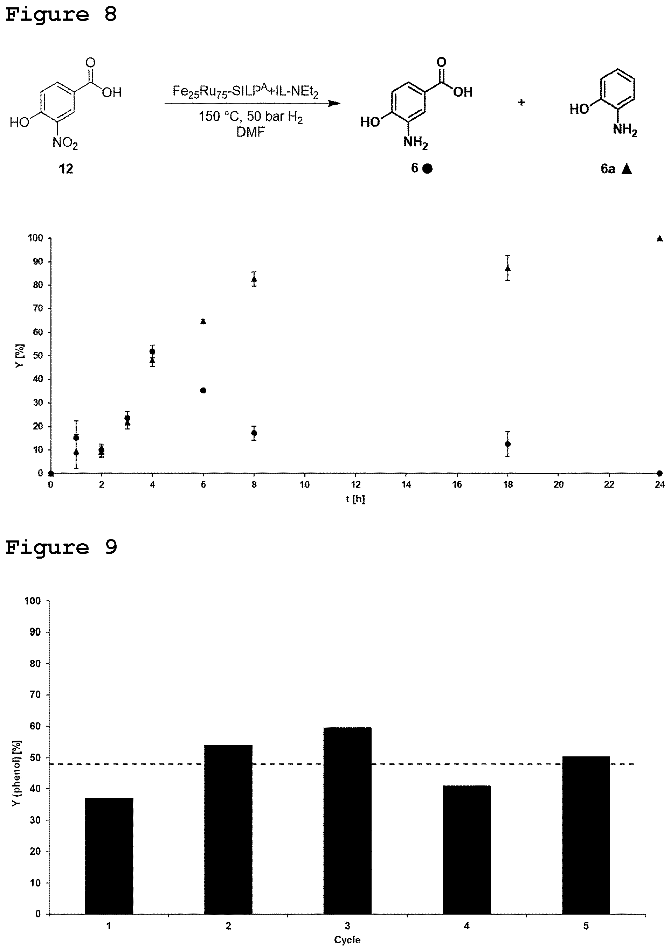

To exploit the capacity of hydrogen activation and transfer at the FeRu NPs of the Fe₂₅Ru₇₅-SILP^{A}+IL-NEt₂ catalyst for tandem reduction/decarboxylation, hydroxy- and aminobenzoic acid derivatives possessing additional reducible functional groups were investigated. Under standard conditions, 3-acetyl-4-hydroxybenzoic (10) was selectively decarboxylated and hydrodeoxygenated directly to produce 2-ethylphenol (10a) in >99% yield (Table 6, Entry 1). Following the reduction/decarboxylation for the nitro-substituted 12 as function of time clearly showed the sequential tandem reaction via 3-amino-4-hydroxybenzoic acid (6) as intermediate (Figure 8). The reaction occurs smoothly to produce selectively 2-aminophenol (6a) in nearly quantitative yield (>99%) yield after 24 h.

Adaptation of the standard reaction conditions (temperature, substrate equivalent, solvent) to ensure good solubility and to limit side-reactions allowed efficient conversion of a range of 2- and 4-hydroxybenzoic acid derivatives possessing acetyl, octanoyl, formyl or nitro substituents (Substrates 11-16, Table 6 Entries 2-7). The corresponding phenol derivatives (11a-16a) were produced in excellent selectivity and yields (95-99%) and isolated for selected examples. Also in this case, replacing the hydroxyl by an amine functionality in 2-amino-5-acetylbenzoic acid (17) did not influence negatively the catalyst performance, and 4-ethylaniline (17a) was obtained in 89% yield (Table 6 Entry 8). Interestingly, many of the products formed are valuable chemicals with widespread applications. In particular, 10a, 6a and 17a are building blocks used in the pharmaceutical industry for the synthesis of anti-tuberculose drugs, anticancer agents and fungicides, respectively. 14a is widely used in the production of non-ionic surfactants.

**Table 6: Decarboxylation and selective reduction of aromatic carboxylic acids**

| 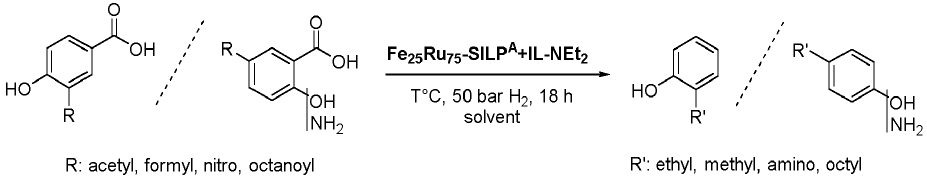 | | | | | | | | |
|---|---|---|---|---|---|---|---|---|
| # | **Substrate** | **Product** | **Solvent** | **Sub:Am** | **Sub:Met** | **T °C** | **X %** | **Y %** |
| 1 | 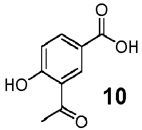 | 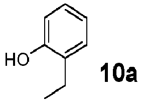 | heptane | 30 | 65 | 175 | >99 | >99 (39) |
| 2 | 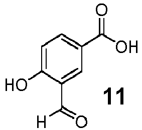 | 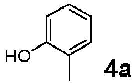 | heptane | 15 | 33 | 200 | >99 | >99 |
| 3 | 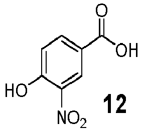 | 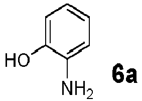 | heptane | 30 | 65 | 150 | >99 | >99 (77) |
| 4 | 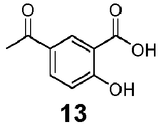 | 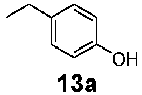 | heptane | 15 | 33 | 175 | >99 | >99 (33) |
| 5 | 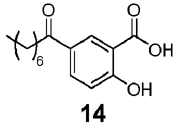 | 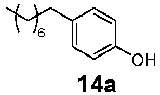 | decalin | 15 | 33 | 200 | >99 | >99 (85) |
| 6 | 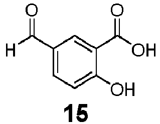 | 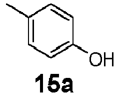 | dioxane | 15 | 33 | 200 | >99 | >99 |
| 7 | 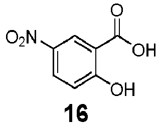 | 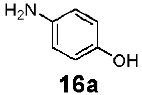 | dioxane | 15 | 33 | 200 | >99 | 95^{[a]} |
| 8 | 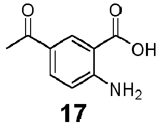 | 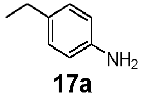 | mesityle ne | 15 | 33 | 200 | >99 | 89^{[b]} |

| | | | | | | | | |
|---|---|---|---|---|---|---|---|---|
| Reaction conditions: Catalyst (10 mg, metal content: 0.0034 mmol), solvent: heptane (0.5 mL), H₂ (50 bar), 18 h, 500 rpm. ^{[a]} 11% aniline. ^{[b]} by-products: dimers. Y: Yield, X: Conversion, determined by GD-FID using tetradecane as internal standard. Isolated yields in parenthesis. | | | | | | | | |

### Example 6: Recycling of the Catalyst

Since leaching of physisorbed ionic liquids is a well-known issue in solution phase catalysis, the stability of Fe₂₅Ru₇₅-SILP^{A}+IL-NEt₂ was carefully studied through recycling experiments using 4-hydroxybenzoic acid (1) as a model substrate (Figure 9). Between each cycle, a sample of the reaction mixture was taken and the catalyst was washed with mesitylene. The reaction time was set to 6 h to ensure an incomplete conversion of the substrate and allow observing any change in activity or selectivity upon recycling. The yield associated to the formation of phenol was fairly constant (48% in average) for at least five cycles without any make-up or regeneration. The fluctuations observed are presumably due to the recycling procedure adopted, during which traces of products may have stayed on the catalyst between each cycle (see below for detailed recycling procedure). Characterization of the catalyst with quantitative SEM-EDS after five cycles did not evidence significant leaching of the metals nor of the physisorbed ionic liquid (Table 7). TEM analysis showed no significant changes in nanoparticle size. N₂ adsorption experiments indicated that the textural properties (BET surface area, pore size, pore volume) of Fe₂₅Ru₇₅-SILP^{A}+IL-NEt₂ were conserved. Titration of the available amine functionalities evidenced a similar loading as on the fresh catalyst, confirming the absence of leaching of the IL-NEt₂ under reaction conditions (Table 7).

**Table 7: Characterisation of the Fe₂₅Ru₇₅-SILP^{A} + IL-NEt₂ catalyst before and after catalysis**

| | **Before catalysis** | **After catalysis (5 cycles)** |
|---|---|---|
| **Accessible amines^{[a]} [%]** | 0.48 | 0.49 |
| **Surface area [m^{2.}g⁻¹]** | 36 ± 1 | 35 ± 1 |
| **Pore volume [m^{3.}g⁻¹]** | 0.13 ± 0.002 | 0.14 ± 0.001 |
| **Metal loading^{[a]} [mmol^{.}g⁻¹]** | 0.34 ± 0.02 | 0.32 ± 0.05 |
| **Metal ratio Fe : Ru^{[a]} [%]** | 27 ± 3 : 73 ± 4 | 22 ± 7 : 78 ± 4 |
| **Particle size [nm]** | 3.1 ± 0.7 | 2.5 ± 0.5 |

| | | |
|---|---|---|
| NP sizes were determined by TEM. Metal ratio and loading were determined using SEM-EDX (Fe-K / Ru-L). ^{[a]} The average values and standard deviations were determined through the characterization of three batches of each material. | | |

### Detailed recycling procedure:

The substrate (33 eq. with respect to metal, 0.11 mmol), catalyst (20 mg, containing 0.0068 mmol metal) and solvent (mesitylene, 0.5 mL) were weighed inside of a glovebox into the autoclave.

The autoclave was closed, transferred out of the glovebox and pressurized with 50 bar hydrogen. After heating the autoclave under stirring (500 rpm) to 200 °C for 6 h, it was cooled down in a water bath and depressurised. The glass insert was taken out and transferred to a Schlenk tube.

The tube was evaporated carefully until the first bubbles from the evaporating solvent show up. Then it was backfilled with argon. This procedure was performed five times and the Schlenk tube with the reaction mixture was transferred into the glovebox. Mesitylene (1 mL) was added to the reaction mixture to solute the product. The supernatant was taken out with a syringe to prepare a GC sample and the catalyst was washed three times with fresh mesitylene. Since the substrate is not soluble in mesitylene, it remained in the autoclave vial. To the washed catalyst were added new substrate (the amount, which was converted in the cycle before) and solvent. The autoclave was closed, pressurised and heated like before to start the new cycle.

Tetradecane (internal standard, 20 mg) and acetone (200 mg) were added to the recovered supernatant to prepare the GC sample.

## Claims

1. A catalyst comprising:
(i) the following structure **I:**
Su - L - Sp - C¹ - R - R'
wherein:
Su is a support;
L is a linker group;
Sp is a spacer group;
C¹ is a cation;
R is an alkylene group with 1-20 carbon atoms,
optionally containing one or more groups selected from ether, ester, amido or arylene groups; and R' is -CH₃ or D, wherein D is a basic group;
wherein structure **I** further comprises a counterion A¹ which is an anion;
and
(ii) the following structure **II:**
CH₃ - R^{a} - C² - R^{b} - D
wherein R^{a} and R^{b} are each independently an alkylene group with 1-20 carbon atoms, optionally containing one or more groups selected from ether, ester, amido or arylene groups; C² is a cation; and D is a basic group;
wherein structure **II** further comprises a counterion A² which is an anion.

2. A catalyst according to claim 1, further comprising bimetallic nanoparticles that are immobilised on the support Su, wherein the bimetallic nanoparticles are represented by:
M¹ₓM²₁₀₀₋ₓ
wherein:
M¹ is a 3d transition metal;
M² is a noble metal; and
x is a number between 1 and 99, preferably between 10 and 80.

3. Catalyst according to claim 1 or 2, wherein the spacer Sp is an alkylene group with 1-30 carbon atoms, optionally containing one or more groups selected from ether, ester, amido or arylene groups;
preferably the spacer Sp is an alkylene group with 2-10 carbon atoms.

4. Catalyst according to any one of claims 1-3, wherein the basic group is an amine, preferably the amine is -NR¹⁴R¹⁵, wherein R¹⁴ and R¹⁵ are each independently H, methyl, ethyl, propyl, or butyl, more preferably R¹⁴ and R¹⁵ are ethyl.

5. Catalyst according to any one of claims 2-4, wherein M¹ is Mn, Fe, Co, Ni or Cu and M² is Rh, Ru, Pd or Pt; preferably M¹ is Fe and M² is Ru.

6. Use of a catalyst according to any one of claims 1-5 in a decarboxylation reaction.

7. Use of a catalyst according to any one of claims 1-5 in a tandem reduction and decarboxylation reaction.

8. Method of decarboxylating a substrate according to formula **IIIa, IIIb, IIIc, IIId,** or **IIIe,** wherein the method comprises bringing the substrate into contact with a catalyst comprising bimetallic nanoparticles and an ionic liquid with a basic group, and heating under pressure in the presence of hydrogen 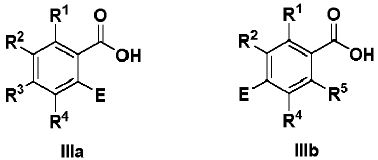 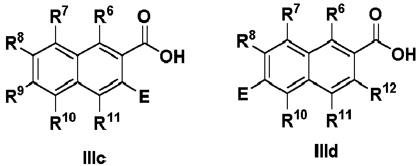 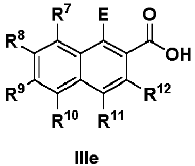 wherein:
E is -OH, -NH₂ or -NR¹³₂, wherein R¹³ is an alkyl group with 1-6 carbon atoms, preferably R¹³ is 1;
preferably wherein E is -OH, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
R¹⁶ is an alkyl group with 1-30 carbon atoms, preferably 1-10 carbon atoms;
the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
the linear or branched alkyl group contains at least one group selected from C-C double bond, C-C triple bond, keto group, ester group, ether group or amido group;
preferably R¹ to R¹² are each independently -H, -OMe, -Me, -F, -NH₂, -OH, -NO₂, -COCH₃, -CHO, or - CO- (CH₂)₆-CH₃.

9. Method of decarboxylating a substrate according to formula **IIIa, IIIb, IIIc, IIId,** or **IIIe,** wherein the method comprises bringing the substrate into contact with a catalyst according to any one of claims 1-5, and heating under pressure in the presence of hydrogen 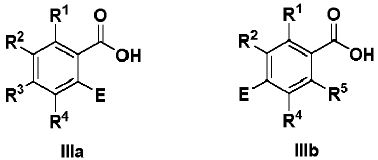 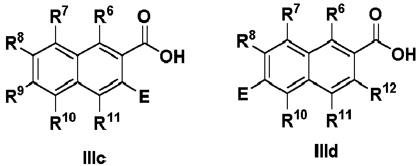 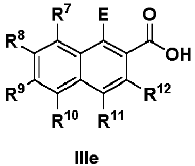 wherein:
E is -OH, -NH₂ or -NR¹³₂, wherein R¹³ is an alkyl group with 1-6 carbon atoms, preferably R¹³ is 1;
preferably wherein E is -OH, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
R¹⁶ is an alkyl group with 1-30 carbon atoms, preferably 1-10 carbon atoms;
the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
the linear or branched alkyl group contains at least one group selected from C-C double bond, C-C triple bond, keto group, ester group, ether group or amido group;
preferably R¹ to R¹² are each independently -H, -OMe, -Me, -F, -NH₂, -OH, -NO₂, -COCH₃, -CHO, or - CO- (CH₂)₆-CH₃.

10. Method according to claim 8 or 9, wherein the substrate is a substrate according to formula **IIIa** or **IIIb,** wherein:
E is -OH or -NH₂; and
R¹ to R⁴ are each independently -H, -OMe, -Me, -F, -NH₂, -OH, -NO₂, -COCH₃, -CHO, or -CO-(CH₂)₆-CH₃.

11. Method of tandem decarboxylation and reduction of a substrate according to formula **IIIa, IIIb, IIIc, IIId,** or **IIIe,** wherein the method comprises bringing the substrate into contact with a catalyst comprising metal nanoparticles and an ionic liquid with a basic group, and heating under pressure in the presence of hydrogen 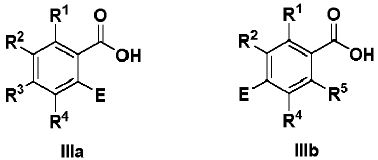 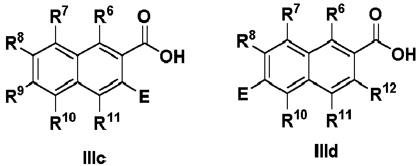 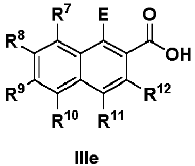 wherein:
E is -OH, -NO₂, -NH₂ or -NR¹³₂, wherein R¹³ is an alkyl group with 1-4 carbon atoms, preferably R¹³ is 1;
preferably wherein E is -OH, -NO₂, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
R¹⁶ is an alkyl group with 1-30 carbon atoms, preferably 1-10 carbon atoms;
the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
the linear or branched alkyl group contains at least one group selected from C-C double bond, C-C triple bond, keto group, ester group, ether group or amido group;
preferably R¹ to R¹² are each independently -H, -OMe, -Me, -F, -NH₂, -OH, -NO₂, -COCH₃, -CHO, or - CO- (CH₂)₆-CH₃.

12. Method of tandem decarboxylation and reduction of a substrate according to formula **IIIa, IIIb, IIIc, IIId,** or **IIIe,** wherein the method comprises bringing the substrate into contact with a catalyst according to any one of claims 2-5, and heating under pressure in the presence of hydrogen 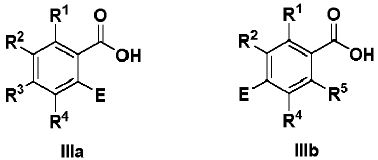 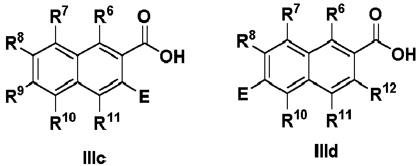 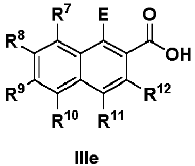 wherein:
E is -OH, -NO₂, -NH₂ or -NR¹³₂, wherein R¹³ is an alkyl group with 1-4 carbon atoms, preferably R¹³ is 1;
preferably wherein E is -OH, -NO₂, or -NH₂;
R¹ to R¹² are each independently -H, -OMe, -Me, -F, -NH₂, -Cl, -Br, -I, -CF₃, -CHO, -NO₂, -NR¹⁶₂, -CN, - OH, or a linear or branched alkyl group, wherein:
R¹⁶ is an alkyl group with 1-30 carbon atoms, preferably 1-10 carbon atoms;
the linear or branched alkyl group is optionally substituted with at least one substituent selected from the group consisting of - OMe, -Me, -F, -NH₂, -Cl, -Br, -I, -CF₃, -CHO, -NO₂, -NR¹⁶₂, -CN, -OH; and/or
the linear or branched alkyl group contains at least one group selected from C-C double bond, C-C triple bond, keto group, ester group, ether group or amido group;
preferably R¹ to R¹² are each independently -H, -OMe, -Me, -F, -NH₂, -OH, -NO₂, -COCH₃, -CHO, or - CO- (CH₂)₆-CH₃.

13. Method according to claim 11 or 12, wherein the substrate is a substrate according to formula **IIIa** or **IIIb,** and wherein:
E is -OH, -NO₂ or -NH₂; and
R¹ and R⁴ are each independently -H, -OMe, -Me, -F, -NH₂, -OH, -NO₂, -COCH₃, -CHO, or -CO-(CH₂)₆-CH₃.

14. Method according to any one of claims 8-13, wherein the substrate is heated at 80-250 °C, preferably 125-200 °C, more preferably 150-200 °C.

15. Method according to any one of claims 8-14, wherein the pressure is 10-100 bar, preferably 20-60 bar.
